# EUROPÄISCHE PATENTSCHRIFT

(11) **EP 2 877 227 B1**
(45) Veröffentlichungstag und Bekanntmachung des Hinweises auf die Patenterteilung: **26.02.2020**
(21) Anmeldenummer: 13745009.4
(22) Anmeldetag: 24.07.2013
(51) Int. Cl.: A61M 25/06

(54) **KANÜLE**
CANNULA
CANULE

(30) Priorität: 25.07.2012 DE 102012106753
(43) Veröffentlichungstag der Anmeldung: 03.06.2015
(60) Teilanmeldung: 15189616.4
(73) Patentinhaber: sfm medical devices GmbH, 63607 Wächtersbach (DE)
(72) Erfinder: HENNINGER, Peter, 63636 Brachttal (DE); HAINDL, Hans, 30974 Wennigsen (DE)
(74) Vertreter: Stoffregen, Hans-Herbert
(86) Internationale Anmeldenummer: PCT/EP2013/065568
(87) Internationale Veröffentlichungsnummer: WO 2014/016322

(56) Entgegenhaltungen:
- US-A- 3 610 239
- US-A- 4 345 596
- US-A- 5 295 977

## Beschreibung

Die Erfindung bezieht sich auf eine Kanüle, insbesondere suprapubische Kanüle zum Einführen eines Katheters in einen Körperhohlraum, mit einem ersten Kanülenabschnitt in Form eines längsgeschnittenen ersten Hülsenkörpers, insbesondere mit im Schnitt eines Kreisbogens oder einer kreisbogenförmigen Geometrie, und einem beim Einführen eines Katheters mit diesem verbundenen zweiten Kanülenabschnitt in Form eines längsgeschnittenen zweiten Hülsenkörpers, insbesondere mit im Schnitt einer Kreisbogen-Geometrie oder einer kreisbogenförmigen Geometrie, wobei einer der Kanülenabschnitte im distalen Ende eine Punktionsspitze aufweist.

Bei z. B. einer Katheterdrainage der Harnblase wird eine Kanüle oberhalb des Schambeins senkrecht zur Bauchdecke bis in die Harnblase gesetzt, um sodann durch die Kanüle einen Katheter einzuführen. Anschließend wird die Kanüle herausgezogen. Da sich diese jedoch nicht über den Ansatz des Katheters ziehen lässt, nimmt man spaltbare Kanülen. Eine solche ist z. B. der DE 42 00 255 A1 zu entnehmen. Die Kanüle weist zwei diametral zueinander verlaufende Sollbruchlinien auf, wobei jede Hälfte der Kanüle einen Griff aufweist, um diese in entgegengesetzte Richtungen zu ziehen, wodurch sich die Kanüle in ihrer Längsrichtung trennt. Zum Trennen der Kanüle bedarf es erheblicher Kraft, so dass für den Anwender ein Verletzungsrisiko besteht. Auch weisen die Ränder scharfe Grate auf, die ein Verletzungsrisiko hervorrufen. Gleiches gilt in Bezug auf die Punktionsspitze, die bei unachtsamer Handhabung der Kanüle zu Verletzungen führen kann.

Eine Kanüle der eingangs genannten Art ist der DE 33 47 150 C1 zu entnehmen. Die Kanüle besteht aus zwei gegeneinander verdrehbaren hülsenartigen Kanülenabschnitten mit Längsschlitz, welche also im Schnitt eine Kreisbogen-Geometrie bzw. kreisbogenförmige Geometrie aufweisen. Die Kanülenabschnitte werden zum Entfernen des Katheters derart zueinander ausgerichtet, dass die Längsschlitze zueinander fluchten. Da die Kanülenabschnitte unter hoher Spannung stehen, um insbesondere gegeneinander abgedichtet zu sein, müssen ggfs. hohe Drehmomente aufgebracht werden, um die Kanülenabschnitte gegeneinander zu verdrehen. Hierdurch bedingt kann eine unkontrollierte Bewegung mit der Folge auftreten, dass der Patient oder der Nutzer durch die Punktionsspitze verletzt wird.

Die DE 10 2011 009 482 A1 bezieht auf eine Sicherheitskanüle, die aus einer äußeren und einer inneren längsgeschnittenen Hülse besteht, wobei die innere Hülse eine Punktionsspitze aufweist. Um zum Beispiel einen innerhalb der Innenhülse verlaufenden Katheterschlauch zu entfernen, werden die Längsschlitze der inneren und äußeren Hülse fluchtend zueinander ausgerichtet. Die Punktionsspitze verläuft innerhalb der äußeren Hülse, so dass eine Verletzungsgefahr vermieden werden kann. Ferner ist vorgesehen, dass sowohl von der inneren als auch von der äußeren Hülse jeweils eine Handhabe ausgeht, die derart betätigt werden können, dass zunächst ein axiales Verschieben der Hülsen zueinander und sodann ein Drehen erfolgt.

Eine spaltbare Kanüle ist der US 4 345 596 A1 zu entnehmen. Die Kanüle wird durch Rohrhälften gebildet, die mittels einer Verzahnung dichtend aneinander liegen. Im proximalen Bereich sind Handhaben vorgesehen, die in Längsrichtung verlaufende Schrägen aufweisen, um beim Längsverschieben der Handhaben die Kanülenhälften voneinander zu trennen.

Die US 5 295 977 A1 bezieht sich auf einen Trokar, dessen Spitze über ein Schutzelement abdeckbar ist.

Siehe auch US 3 610 239 A.

Der vorliegenden Erfindung liegt die Aufgabe zu Grunde, eine Kanüle der eingangs genannten Art so weiterzubilden, dass ein problemloses Trennen der Kanülenabschnitte ermöglicht wird. Nach einem weiteren Aspekt der Erfindung soll sichergestellt sein, dass das Risiko einer Verletzung durch die Punktionsspitze vermieden wird.

Zur Lösung zumindest eines Aspekts der Erfindung ist vorgesehen, dass sich die miteinander verbundenen Kanülenabschnitte umschlingen, wobei der erste Kanülenabschnitt einen Umschlingungswinkel in Bezug auf den zweiten Kanülenabschnitt aufweist, der in einem senkrecht zur Längsachse des ersten Kanülenabschnitts verlaufenden Schnitt größer als 180° ist, und dass zum Lösen der Kanülenabschnitte voneinander einer der Kanülenabschnitte gegenüber dem anderen Kanülenabschnitt mittels zumindest eines von einem der Kanülenabschnitte ausgehenden und sich direkt oder indirekt an dem anderen Kanülenabschnitt abstützenden

Betätigungselements bei Krafteinwirkung auf das Betätigungselement quer zur Längsachse der Kanüle abziehbar ist, wobei das Betätigungselement ein Hebelelement ist, das über einen von diesem abragenden Vorsprung an einem Abschnitt eines von dem anderen Kanülenabschnitt ausgehenden Abstützelements abstützbar ist. Abweichend von vorbekannten Kanülen, die aus zwei hülsenartigen Kanülenabschnitten mit Längsschlitz bestehen, werden nach der erfindungsgemäßen Lehre die ebenfalls hülsenartigen Kanülenabschnitte mit Längsschlitz der Kanüle gemäß Anspruch 1 quer zur Längsachse auseinander gezogen, wobei eine kontrollierte Krafteinwirkung zum Lösen bzw. Beabstanden der Kanülenabschnitte mittels eines Betätigungselementes erfolgt, das von einem Kanülenabschnitt ausgeht und sich direkt oder indirekt an dem anderen Kanülenabschnitt abstützt, um die Trennung durchzuführen.

Insbesondere ist vorgesehen, dass bei Krafteinwirkung des Betätigungselementes in Längsrichtung der Kanüle, vorzugsweise in Richtung des distalen Bereichs der Kanüle, der Kanülenabschnitt, von dem das Betätigungselement ausgeht, zunächst in Längsrichtung der Kanüle entlang des anderen Kanülenabschnitts verschiebbar ist und dass sodann bei weiterer Krafteinwirkung der eine Kanülenabschnitt zumindest im proximalen Bereich von dem anderen Kanülenabschnitt lösbar ist.

Das Betätigungselement ist ein Hebelelement, das über einen von diesem abragenden Vorsprung an einem insbesondere rampenförmigen Abschnitt des Abstützelements abstützbar ist.

Dabei sollte insbesondere das Abstützelement ein sich quer zur Längsrichtung des zweiten Kanülenabschnitts erstreckendes plattenförmiges Element sein, das in Richtung des Lösens der Kanülenabschnitte voneinander den ersten Kanülenabschnitt in einem Abstand umgibt, der ein Lösen der Kanülenabschnitte ermöglicht.

Nach einem eigenerfinderischen Vorschlag und zur Lösung eines weiteres Aspektes der Erfindung ist vorgesehen, dass von dem ersten Kanülenabschnitt einer Kanüle gemäß Anspruch 1 ein entlang dessen Innenseite verstellbares Schutzelement ausgeht, das in Nutzposition der Kanüle beabstandet zu der Punktionsspitze verläuft und nach Trennen der Kanülenabschnitte die Punktionsspitze abdeckt. Das Schutzelement ist derart zu dem die Punktionsspitze aufweisenden Kanülenabschnitt verschiebbar, dass dann, wenn der die Punktionsspitze aufweisende Kanülenabschnitt senkrecht zu dem zweiten Kanülenabschnitt verschoben wird, das Schutzelement in Richtung der Punktionsspitze zum Abdecken dieser verstellt wird.

Dabei ist bevorzugterweise vorgesehen, dass das Schutzelement ein zungen- oder rinnenförmiges Element ist, in dessen Längsrichtung ein Schlitz verläuft, von dessen Längsrandseiten flügelartige Abschnitte ausgehen, dass von dem ersten Kanülenabschnitt nach innen umgebogene und den Schlitz durchsetzende und an dessen Längsrandabschnitten anliegende Halteabschnitte ausgehen, die in Längsrichtung des ersten Kanülenabschnitts eine Länge aufweisen, die kürzer als die Länge des Schlitzes ist, und dass in Nutzposition der Kanüle auf den Längsrändern der flügelartigen Abschnitte die Längsränder des zweiten Kanülenabschnitts bereichsweise aufliegen. Ferner weist der Kanülenabschnitt in seinem distalen Bereich eine stufenförmige Aussparung auf, die sich an den flügelartigen Abschnitten abstützt, um die gewünschte Relativbewegung zwischen dem Schutzelement und dem ersten Kanülenabschnitt zu ermöglichen mit der Folge, dass das Schutzelement über die Punktionsspitze hinausgeschoben wird. Das Abdecken der Punktionsspitze erfolgt über einen entsprechend abgewinkelten Abschnitt des distalen Endes des Schutzelementes. Es besteht jedoch auch die Möglichkeit, dass das Schutzelement in seinem distalen Bereich eine Aussparung aufweist, in die die nach innen gebogene Punktionsspitze zum Verrasten des Schutzelementes eindringt.

Dadurch, dass die Längsränder des zweiten Kanülenabschnitts bereichs- bzw. abschnittsweise auf den Längsrändern der flügel artigen Abschnitte aufliegen, ist des Weiteren sichergestellt, dass ein unkontrolliertes Hineindrücken des zweiten Kanülenabschnitts in den ersten Kanülenabschnitt unterbunden wird.

Um sicherzustellen, dass die flügelartigen Abschnitte ein Lösen der Kanülenabschnitte voneinander nicht behindern, ist vorgesehen, dass der zweite Kanülenabschnitt in Verschieberichtung der flügelartigen Abschnitte zumindest eine Aussparung aufweist, in die beim Lösen des ersten Kanülenabschnitts von dem zweiten Kanülenabschnitt die flügelartigen Abschnitte eingreifen.

Nach einer alternativen Lösung zur Ausbildung des Schutzelementes wird vorgeschlagen, dass der erste Kanülenabschnitt in dessen Längsrichtung verlaufende Langlöcher aufweist, in die von dem Schutzelement ausgehende Vorsprünge eingreifen, dass der zweite Kanülenabschnitt mit dem Schutzelement derart wechselwirkt, dass bei Längsverschiebung des ersten Kanülenabschnitts zu dem zweiten Kanülenabschnitt das Schutzelement in Richtung der Punktionsspitze und zum Abdecken dieser verschiebbar ist.

Um bei der diesbezüglichen Ausführungsform die Relativbewegung zwischen dem Schutzelement und dem die Punktionsspitze aufweisenden ersten Kanülenabschnitt sicherzustellen, um also das Schutzelement über die Punktionsspitze zu verschieben, ist insbesondere vorgesehen, dass das Schutzelement proximal einen stufenförmigen Abschnitt aufweist, an dem der distale Bereich des zweiten Kanülenabschnitts bei Verschieben des ersten Kanülenabschnitts zu dem zweiten Kanülenabschnitt anliegt.

Das Schutzelement kann z. B. ein Kunststoffteil wie Spritzgussteil sein, aber auch aus Metall bestehen. Wird ein Kunststoffteil benutzt, so verhakt dieses mit der Punktionsspitze dann, wenn diese geringfügig in Richtung der Längsachse nach innen gebogen ist, so dass das Schutzelement bleibend die Punktionsspitze abdeckt. Hierzu ist vorzugsweise der distale Bereich des Schutzelementes U-förmig abgewinkelt, so dass die Punktionsspitze insbesondere zwischen freiem Seitenschenkel und Querschenkel des distalen Abschnitts des Schutzelementes fixiert wird.

Wird ein Schutzelement aus einem gebogenen Metallstreifen benutzt, so sollte das Schutzelement in seinem distalen Bereich eine Aussparung aufweisen, in die bei abgedeckter Punktionsspitze diese eingreift. Auch kann der distale Bereich des aus Metall bestehenden Schutzelementes hakenförmig, also im Schnitt in etwa U-förmig gebogen sein, um sicherzustellen, dass die Spitze in diesem Bereich verbleibt und ein unkontrolliertes Lösen des Schutzelementes verhindert wird.

Um bei einem Metallteil ein unkontrolliertes Lösen des Schutzelementes von der Punktionsspitze auszuschließen, um also den distalen Bereich des Schutzelements in Richtung der Kanülenspitze kraftzubeaufschlagen, ist vorgesehen, dass das Schutzelement in seinem proximalen Bereich ein sich an der Innenwandung des ersten Kanülenabschnitts abstützendes Zungenelement zur Krafteinleitung in den distalen Bereich des Schutzelements in Richtung der Innenseite des ersten Kanülenabschnitts aufweist.

Eine Kanüle zum Einrühren eines Katheters in einen Körperhohlraum, wobei die Kanüle nach dem Ablegen des Katheters von dem Katheter getrennt werden soll, muss nicht zwingend zwei Kanülenabschnitte aufweisen, die eine zuvor beschriebene Gestaltung aufweisen, die auch als geschnittene Hülsenkörper bezeichnet werden können. Vielmehr kann eine Kanüle - nicht zum Gegenstand der Erfindung gehörend - benutzt werden, die einen einzigen hülsenförmigen längsgeschnittenen Körper aufweist, wobei die Hülse, die auch als Schale bezeichnet werden kann, im Schnitt einen Bogen zwischen 200° und 270° aufweisen sollte, wenn man von einem Querschnitt ausgeht, der einem Kreisbogen folgt.

Auch für entsprechende einschalige Kanülen ist nach einem Aspekt der vorliegenden Lehre die Möglichkeit gegeben, dass die Punktionsspitze derart abgedeckt wird, dass eine Verletzungsgefahr unterbunden wird. Abdecken bedeutet dabei, dass die Punktionsspitze mit einem Schutz versehen ist, der es ermöglicht, dass die Punktionsspitze nicht freiliegt.

Die Offenbarung bezieht sich daher auch auf eine Kanüle mit in dessen Längsrichtung verschiebbarem Katheter, insbesondere suprapubische Kanüle zum Einführen eines Katheters in einen Körperhohlraum, umfassend zumindest einen ersten Kanülenabschnitt, wie längsgeschnittenen Hülsenkörper, insbesondere mit im Schnitt eines Kreisbogens oder einer kreisbogenförmigen Geometrie, mit einem proximalen Ende und einem distalen Ende mit Punktionsspitze und zeichnet sich dadurch aus, dass von der Innenseite der Kanüle ein verstellbares Schutzelement ausgeht, das in Nutzposition der Kanüle beabstandet zu der Punktionsspitze verläuft und nach Verschieben des Katheters in Richtung der Punktionsspitze und/oder bei von dem ersten Kanülenabschnitt aufgenommenen zweiten Kanülenabschnitt mit im Schnitt eines Kreisbogens oder einer kreisbogenförmigen Geometrie und Längsverschiebung des ersten Kanülenabschnitts zu dem zweiten Kanülenabschnitt die Punktionsspitze von dem Schutzelement abgedeckt und/oder das Schutzelement von der Punktionsspitze bereichsweise durchsetzt ist.

Insbesondere ist vorgesehen, dass das Schutzelement mit der Punktionsspitze und/oder in dem ersten Kanülenabschnitt verrastet.

Insbesondere ist vorgesehen, dass das Schutzelement mit der Kanüle verbunden wie verschweißt, verklebt oder vernietet ist und bei fehlender Einwirkung des Katheters auf das Schutzelement einen innerhalb der Kanüle in Richtung der Kanülenlängsachse verlaufenden Bogenabschnitt aufweist, wobei distales Ende des Schutzelementes beabstandet zu der Punktionsspitze verläuft, und dass bei Verstellen des Katheters in Richtung der Punktionsspitze und Wechselwirken mit dem Schutzelement der gebogene Abschnitt in Richtung der Kanüleninnenseite gedrückt wird derart, dass das Schutzelement durch scheinbare Längenveränderung mit seinem distalen Ende mit der Punktionsspitze wechselwirkt.

Insbesondere kann das Schutzelement in seinem vorzugsweise zungenförmig ausgebildeten distalen Bereich eine Öffnung aufweisen, in die bei in Richtung der Kanüleninnenseite gedrücktem Schutzelement die Punktionsspitze eingreift. Das Schutzelement kann in seinem distalen Bereich auch einen Geometrieverlauf wie den oder in etwa den des distalen Stirnrandes der Kanüle bzw. des ersten Kanülenabschnitts aufweisen.

Es besteht jedoch auch die Möglichkeit, dass das Schutzelement in seinem distalen Bereich eine Aufnahme für die Punktionsspitze aufweist. Dabei kann die Aufnahme z. B. durch ein oder mehrere gebogene Randabschnitte des distalen Endes des Schutzelementes gebildet sein, so dass sich eine quasi taschenförmige Aufnahme für die insbesondere nach innen, also in Richtung Kanülenlängsachse gebogene Punktionsspitze ergibt.

Nach einer weiteren Ausgestaltung der Offenbarung ist vorgesehen, dass das Schutzelement mit dem Hülsenkörper in seinem zum distalen Ende fernliegenden Bereich, also insbesondere im proximalen Bereich nicht fest verbunden ist, sondern insgesamt in Längsrichtung des Hülsenkörpers verschiebbar ist. Hierzu ist insbesondere vorgesehen, dass der Hülsenkörper in Längsrichtung verlaufende Schlitze aufweist, in die Abschnitte des Schutzelementes eingreifen. Zum Verstellen des Schutzelementes weist dieses einen ins Innere der Kanüle ragenden Abschnitt auf, der beim Verstellen des Katheters mitgenommen wird, so dass der distale Bereich des Schutzelementes in den Bereich der Punktionsspitze gelangt und diese abdeckt oder die Punktionsspitze in das Schutzelement eindringt, die eine Öffnung durchsetzen kann.

Eine weitere Gestaltung eines Schutzelementes sieht vor, dass dieses schwenkbar mit der Kanüle verbunden ist derart, dass bei Verstellen des Katheters in Richtung der Punktionsspitze das Schutzelement in Richtung der Punktionsspitze derart verschwenkt wird, dass der distale Bereich des Schutzelementes die Punktionsspitze abdeckt oder diese das Schutzelement durchsetzt.

Die Erfindung zeichnet sich nach einem selbständigen Lösungsvorschlag auch dadurch aus, dass das Schutzelement innerhalb des ersten Kanülenabschnitts an dessen Innnenseite zumindest bereichsweise anliegt und dass der erste Kanülenabschnitt in seinem dem Längsschlitz gegenüberliegenden Bereich eine Aufnahme wie Öffnung aufweist, in die ein von dem Schutzelement ausgehender Vorsprung bei die Punktionsspitze abdeckendem Schutzelement eingreift.

Hierzu wird vorzugsweise vorgeschlagen, dass der in die Aufnahme einrastbare Vorsprung des Schutzelementes durch zwei quer zur Längsachse des Schutzelementes verlaufende aus dem Schutzelement gebogene flügelartige Abschnitte gebildet ist.

Insbesondere ist vorgesehen, dass das Schutzelement vorgespannt zumindest bereichsweise an der Innenseite des ersten Kanülenabschnitts anliegt. Hierdurch ergibt sich der Vorteil, dass das Schutzelement den inneren Querschnitt des Kanülenabschnitts nicht wesentlich verringert, so dass weiterhin ein ungehindertes axiales Verschieben des Katheters möglich ist.

Hervorzuheben ist des Weiteren, dass das Schutzelement mit einem im Schnitt kreisbogenförmigen oder einem Kreisbogen folgenden Abschnitt zumindest bereichsweise an der Innenseite anliegt, dass der Abschnitt parallel zur Längsachse des ersten Kanülenabschnitts verlaufende Längsränder aufweist und dass an den Längsrändern der von dem ersten Kanülenabschnitt aufgenommene zweite Kanülenabschnitt mit seinen Längsrändern zumindest bereichsweise anliegt. Durch diese konstruktiven Maßnahmen ist eine sichere Führung des Schutzelementes in Längsrichtung der Kanüle sichergestellt. Gleichzeitig ergibt sich eine Unverdrehbarkeit.

Unabhängig von der Art, wie das Schutzelement zur Erzielung der Relativbewegung zu der Kanüle bzw. zum ersten Kanülenabschnitt fixiert wird, ist vorgesehen, dass das Schutzelement zungen- oder rinnenförmig ausgebildet ist und außenseitig flächig oder im Wesentlichen flächig an der Innenseite des ersten Kanülenabschnitts bzw. des Hülsenkörpers anliegt.

Im proximalen Bereich sollte der erste Kanülenabschnitt senkrecht zur Längsachse zumindest abschnittsweise ein Bogenmaß B₁ mit B₁ > π und der zweite Kanülenabschnitt ein Bogenmaß B₂ mit B₁ + B₂ > 2 π aufweisen.

Mit anderen Worten ist in einem senkrecht zur Längsachse des ersten Abschnitts verlaufenden Schnitt die Bogenlänge des ersten Kanülenabschnitts größer als R₁ π, wobei R₁ tatsächlicher bzw. fiktiver Radius des ersten Kanülenabschnitts im Schnittbereich ist. Der Umschlingungswinkel zwischen den Längsrändern des ersten Kanülenabschnitts beträgt somit mehr als 180°, liegt insbesondere zwischen 245° und 310°. Der Umschlingungswinkel kann vom distalen Bereich bis zum proximalen Bereich hin abnehmen.

Dabei ist insbesondere vorgesehen, dass senkrecht zur Längsachse der Kanüle der erste Kanülenabschnitt im proximalen Bereich ein Bogenmaß B₃ und der zweite Kanülenabschnitt ein Bogenmaß B₄ mit B₃ < π und B₃ + B₄ > 2π aufweisen.

Insbesondere ist vorgesehen, dass der erste und der zweite Kanülenabschnitt im distalen Bereich, insbesondere bis in den Bereich des Schutzelementes, jeweils ein Bogenmaß B₅ mit B₅ > π, insbesondere 1,2 π < B₅ ≤ 1,75 π aufweist.

Um das Abziehen des ersten oder äußeren Kanülenabschnitts von dem zweiten oder inneren Kanülenabschnitt zu erleichtern, hat es sich als vorteilhaft erwiesen, dass zumindest einer der Kanülenabschnitte, vorzugsweise der erste Kanülenabschnitt auf der zu seiner Längsöffnung gegenüberliegenden Seite durch z. B. Prägen materialgeschwächt ist.

Alternativ oder ergänzend kann vorgesehen sein, dass vom proximalen Rand einer der Kanülenabschnitte wie des ersten Kanülenabschnitts auf der zu dessen Längsöffnung gegenüberliegender Seite ein Schlitz ausgeht und dass von jedem der zu dem Schlitz benachbarten Bereiche des Kanülenabschnitts ein Betätigungselement ausgeht.

Zum Abziehen des äußeren oder ersten Kanülenabschnitts von dem inneren oder zweiten Kanülenabschnitt ist es sodann nur erforderlich, dass nach der Kraftbeaufschlagung der Betätigungselemente in Richtung der Punktionsspitze, wodurch die Relativverschiebung zwischen dem Schutzelement und dem ersten Kanülenabschnitt erfolgt, die Betätigungselemente aufeinanderzu bewegt werden, so dass ein Spreizen der Längsränder des ersten Kanülenabschnitts im proximalen Bereich mit der Folge gegeben ist, dass ein problemloses Abziehen des ersten Kanülenabschnitts von dem zweiten Kanülenabschnitt ohne größere Krafteinleitung möglich ist.

Bestehen vorzugsweise sowohl der erste als auch der zweite Kanülenabschnitt aus Metall, wobei der erste und zweite Kanülenabschnitt durch Rollen eines Blechstreifens hergestellt sein kann, so besteht auch die Möglichkeit, dass der zweite Kanülenabschnitt aus Kunststoff besteht und z. B. ein Spritzgussteil ist.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: einen Schnitt durch eine Kanüle, die einen ersten und einen zweiten Kanülenabschnitt aufweist, die ineinandergreifen,
- Fig. 2: die Kanüle nach Fig. 1, bei der der erste Kanülenabschnitt von dem zweiten Kanülenabschnitt abgezogen wird,
- Fig. 3: eine Draufsicht auf ein Abstützelement und ein Betätigungselement, die vom proximalen Bereich der Kanülenabschnitte ausgehen,
- Fig. 4: einen Schnitt durch die Darstellung gemäß Fig. 3 und im Ausschnitt,
- Fig. 5: einen Schnitt durch die Kanüle gemäß Fig. 1 in deren distalen Bereich mit einer ersten Ausführungsform eines Schutzelementes,
- Fig. 6: einen Schnitt entlang der Linie B-B in Fig. 5,
- Fig. 7: einen Ausschnitt des zweiten Kanülenabschnitts,
- Fig. 8: einen Ausschnitt der Kanüle im distalen Bereich,
- Fig. 9: einen Ausschnitt der Kanüle im distalen Bereich mit einer zweiten Ausführungsform eines Schutzelementes,
- Fig. 10: einen Schnitt durch die Kanüle gemäß Fig. 9 im Bereich des Schutzelementes,
- Fig. 11: eine Schnittdarstellung der Kanüle gemäß Fig. 9 und 10 im distalen Bereich,
- Fig. 12: eine der Fig. 11 entsprechende Darstellung, bei der das Schutzelement verriegelt ist,
- Fig. 13: eine Seitenansicht einer Kanüle,
- Fig. 14: eine Rückansicht einer weiteren Ausführungsform einer Kanüle,
- Fig. 15: eine weitere Kanüle in Seitenansicht,
- Fig. 16: eine weitere Ausführungsform eines Betätigungselements mit Griff in Draufsicht und
- Fig. 17: das Betätigungselement mit Griff gemäß Fig. 16 in aktivierter Position,
- Fig. 18: eine Kanüle mit einer dritten Ausführungsform eines Schutzelementes in Seitenansicht,
- Fig. 19: die Darstellung gemäß Fig. 18 im Schnitt,
- Fig. 20: das den Fig. 18 und 19 zu entnehmende Schutzelement in aktivierter Stellung und in Seitenansicht,
- Fig. 21: die Darstellung gemäß Fig. 20 in Schnittdarstellung,
- Fig. 22: in Seitenansicht eine Kanüle mit einer vierten Ausführungsform eines Schutzelementes,
- Fig. 23: die Darstellung gemäß Fig. 22 in Schnittdarstellung,
- Fig. 24: das Schutzelement gemäß der Fig. 22 und 23 mit Kanüle und Katheter im Ausschnitt und Draufsicht,
- Fig. 25: das Schutzelement der Fig. 22 bis 24 in aktivierter Stellung,
- Fig. 26: das aktivierte Schutzelement gemäß Fig. 25 mit Punktionsspitze,
- Fig. 27: eine Seitenansicht des aktivierten Schutzelementes gemäß der Fig. 22 bis 26 mit Punktionsspitze,
- Fig. 28: eine Seitenansicht einer Kanüle mit einer fünften Ausführungsform eines Schutzelementes,
- Fig. 29: die Darstellung gemäß Fig. 28 in Schnittdarstellung,
- Fig. 30: eine Unteransicht der Kanüle gemäß der Fig. 28 und 29 mit aktiviertem Schutzelement,
- Fig. 31: die Kanüle der Fig. 28 bis 30 mit aktiviertem Schutzelement,
- Fig. 32: die Darstellung gemäß Fig. 31 in Schnittdarstellung,
- Fig. 33: eine vergrößerte Darstellung der Punktionsspitze der Kanüle gemäß der Fig. 28 bis 32 mit aktiviertem Schutzelement,
- Fig. 34: eine Schnittdarstellung einer Kanüle mit einer sechsten Ausführungsform eines Schutzelementes in Ausgangslage,
- Fig. 35: die Kanüle gemäß Fig. 34 mit dem Schutzelement in aktivierter Stellung,
- Fig. 36: einen Schnitt durch eine Kanüle mit einer weiteren Ausführungsform eines Schutzelementes,
- Fig. 37: die Kanüle gemäß Fig. 36 mit aktiviertem Schutzelement,
- Fig. 38: einen Schnitt durch die Kanüle gemäß Fig. 36 und 37,
- Fig. 39: einen Ausschnitt einer Kanüle mit einer achten Ausführungsform eines Schutzelementes,
- Fig. 40: die Kanüle gemäß Fig. 39 mit aktiviertem Schutzelement,
- Fig. 41: einen Ausschnitt einer Kanüle mit einer neunten Ausführungsform eines Schutzelementes und
- Fig. 42: die Kanüle gemäß Fig. 41 mit aktiviertem Schutzelement.

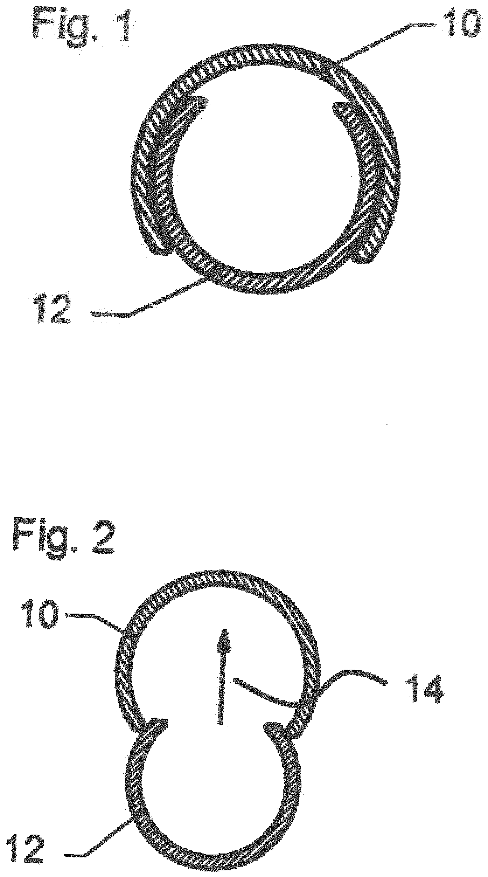

In den Figuren 1 bis 17, in denen grundsätzlich gleiche Elemente mit gleichen Bezugszeichen gekennzeichnet werden, sind zwei Ausführungsformen einer Kanüle dargestellt, die nach Legen eines Katheters von diesem entfernt werden kann, ohne dass die Kanüle über den Ansatz des Katheters geschoben werden muss. Hierzu besteht die Kanüle aus zwei Kanülenabschnitten, und zwar einem äußeren oder ersten Kanülenabschnitt 10 und einem zweiten oder inneren Kanülenabschnitt 12, wobei der erste Kanülenabschnitt 10 den zweiten Kanülenabschnitt 12 umfasst, wie sich aus einem Vergleich der Fig. 1 und 2 ergibt.

Sowohl der erste, also äußere Kanülenabschnitt 10, als auch der zweite, also innere Kanülenabschnitt 12, weisen im Schnitt eine hülsenartige Geometrie mit Längsschlitz, also eine Bogen- bzw. bogenförmige Geometrie auf. Dabei kann jedoch der Umschlingungswinkel des ersten Abschnitts 10 vom distalen zum proximalen Bereich hin abnehmen. Der jeweilige Abschnitt 10, 12 kann auch als Hülsenkörper mit Längsschlitz oder als Schalenkörper bezeichnet werden.

In der idealisierten Bogenform weist der Kanülenabschnitt 10 zumindest im proximalen Bereich der Kanüle ein Bogenmaß B₁ mit B₁ größer π auf, also einen Umschlingungswinkel in Bezug auf den zweiten Kanülenabschnitt 12, der größer als 180° ist, insbesondere im Bereich zwischen 245° und 305° liegt. Der innere Abschnitt 12 kann einen entsprechenden Öffnungswinkel aufweisen, also gleichfalls ein Bogenmaß größer π besitzen. Die Bogenlänge des ersten Abschnitts 10 ist somit größer π · R₁ mit R₁ tatsächlicher oder fiktiver Radius des ersten Kanülenabschnitts 10. Die Bogenlänge des zweiten Kanülenabschnitts 12 in einem senkrecht zur Längsahse der Kanüle verlaufenden Schnitt ist demzufolge gleichfalls größer π · R₂ und mit R₂ tatsächlich oder fiktiver Radius des zweiten Abschnitts 12.

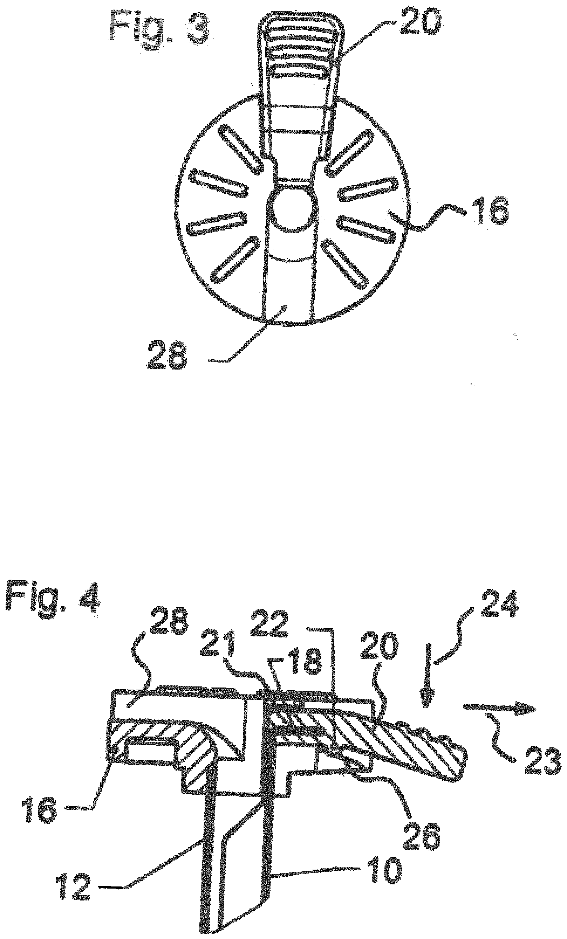

Werden nach dem Stand der Technik entsprechende schalen- oder rinnenförmige Abschnitte einer Kanüle zueinander verdreht, um den Innenraum der Kanüle frei zugänglich zu machen, um also über die zueinander ausgerichteten Schlitze der Abschnitte einen Katheter durchzuführen, ist erfindungsgemäß vorgesehen, dass der erste Kanülenabschnitt 10 zu dem zweiten Kanülenabschnitt 12 quer zur Längsrichtung der Kanüle auseinander gezogen werden, wie durch den Pfeil 14 angedeutet wird. Hierzu ist erfindungsgemäß vorgesehen, dass von proximalem Bereich des zweiten Kanülenabschnitts 12 ein insbesondere tellerförmig ausgebildeter Griff als Abstützelement 16 ausgeht, das sich quer bzw. senkrecht zur Längsachse des zweiten Kanülenabschnitts 12 erstreckt, wie ein Vergleich der Fig. 3 und 4 verdeutlicht. Das proximale Ende des ersten Kanülenabschnitts 10 ist nach außen abgewinkelt oder weist einen entsprechenden abgewinkelten Abschnitt 18 auf. Von diesem geht ein als Hebel 20 ausgebildetes Betätigungselement aus, das über einen Vorsprung 22 auf einen rampenförmig ausgebildeten Abschnitt 26 des Griffs oder Abstützelementes 16 abgestützt ist, so dass infolgedessen das als Hebel ausgebildete Betätigungselement 20 um den auch als Noppen zu bezeichnenden Vorsprung 22 drehbar ist. Aus der Schnittdarstellung der Fig. 4 ist des Weiteren erkennbar, dass der platten- oder tellerförmige Griff im Bereich der rampenförmigen Abschnitte 26 derart ausgeschnitten ist, dass entsprechend der Richtung des Pfeils 14 der erste Kanülenabschnitt 10 zu dem Abstützelement 16 und damit dem zweiten Kanülenabschnitt 12 in einem Umfang verstellbar ist, dass ein Lösen, also ein Abziehen des ersten Kanülenabschnitts 10 von dem zweiten Kanülenabschnitt 12 ermöglicht wird. Gegenüberliegend zu dem Hebel 20 weist das Abstützelement 16 eine Vertiefung 28 zum Einführen des Katheters in die Kanüle auf.

Damit eine Relativverschiebung des ersten und zweiten Kanülenabschnitts 10, 12 beim Setzen der Kanüle unterbleibt, wird der Hebel 20 verriegelt. Hierzu unterfasst der kanülenseitige Bereich des Hebels 20 einen Steg oder Vorsprung 21 des Griffs oder Abstützelements 16, wie die Fig. 4 verdeutlicht. Um ein Lösen von dem nicht dargestellten Katheter vorzunehmen, also die axiale und radiale Verstellung des ersten und zweiten Kanülenabschnitts 10, 12 durchführen zu können, muss sodann zunächst der Hebel 20 in Pfeilrichtung 23 in einem Umfang verstellt werden, dass der Hebel 20 in Ausgriff mit dem Vorsprung bzw. Steg 21 gelangt. Sodann kann der Hebel 20 um den Vorsprung 28 auf dem rampenförmigen Abschnitt 26 des Griffs oder Abstützelementes 16 durch Einleitung einer Kraft in Richtung des Pfeils 24 verschwenkt werden.

Wirkt auf den Hebel 20 eine Kraft in Richtung des Pfeils 24, so wird zunächst der äußere oder erste Kanülenabschnitt 10 in Richtung der Längsachse der Kanüle relativ zu dem zweiten Kanülenabschnitt 12 verschoben, der sodann bei weiterer Krafteinleitung radial zu dem zweiten Kanülenabschnitt 12 von diesem weggezogen wird, so dass entsprechend der Prinzipdarstellung der Fig. 2 der erste Kanülenabschnitt 10 von dem zweiten Kanülenabschnitt 12 quer zur Längsrichtung der Kanüle abgezogen wird. Dies wird beim Betätigen des Hebels 24 spürbar, da dann, wenn die Klemmung zwischen dem ersten und zweiten Kanülenabschnitt 10, 12 überwunden ist, ein leichtes seitliches Abziehen des ersten Kanülenabschnitts 10 von dem zweiten Kanülenabschnitt 12 ermöglicht wird.

Selbstverständlich können die Betätigungselemente, mittels derer der erste und zweite Kanülenabschnitt 10, 12 zunächst axial und sodann radial zueinander verstellbar sind, um ein Abziehen des ersten Kanülenabschnitts von dem zweiten Kanülenabschnitt zu ermöglichen, konstruktiv auch so gelöst werden, dass bei einer Krafteinwirkung vom distalen Bereich der Kanüle weg der entsprechende Bewegungsablauf erfolgt.

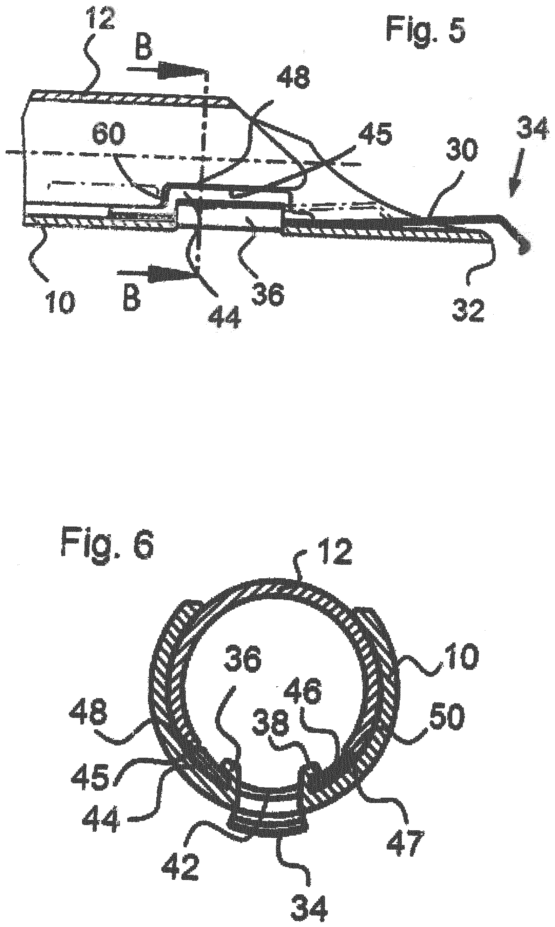

Während der axialen Relativverstellung zwischen dem ersten und zweiten Abschnitt 10, 12 wird gleichzeitig ein Sicherheitsmechanismus im distalen Bereich der Kanüle betätigt, wodurch ein insbesondere zungen- oder rinnenförmiges Schutzelement 30 relativ zu dem ersten Kanülenabschnitt 10 in Richtung der Spitze bzw. Punktionsspitze 32 des ersten Kanülenabschnitts 10 verstellt wird, so dass diese abgedeckt wird und somit eine Verletzungsgefahr ausgeschlossen ist. Hierzu ist das Schutzelement 30 distal abgewinkelt (Bereich 34), so dass dann, wenn der Bereich 34 über der Punktionsspitze 32 vorsteht, die Punktionsspitze 32 hinreichend abgedeckt wird, wie die Schnittdarstellung gemäß Fig. 5 verdeutlicht. Um die Relativverschiebung des Elements 30 beim axialen Verschieben des ersten Abschnitts 10 zu dem zweiten Abschnitt 12 zu ermöglichen, ist nach dem Ausführungsbeispiel der Fig. 5 bis 8 folgende Konstruktion gewählt.

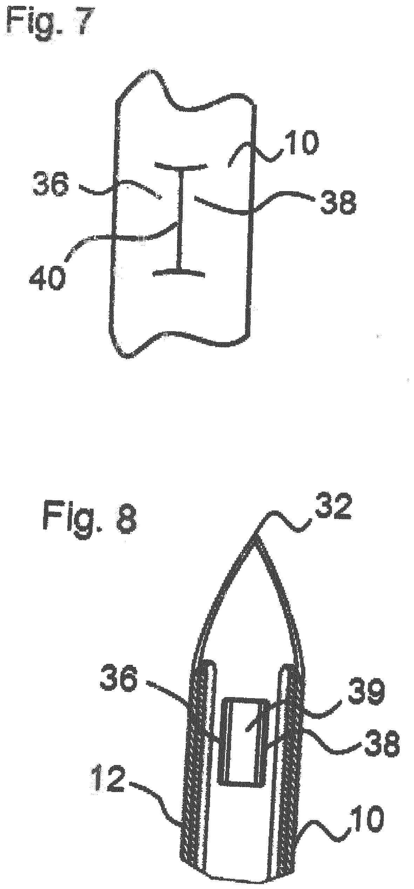

Das eine Zungen- oder Rinnengeometrie aufweisende Schutzelement 30, das flächig an der Innenseite des ersten Kanülenabschnitts 10 anliegen sollte, weist eine rechteckförmige, sich in Längsrichtung des Schutzelementes 30 erstreckende Aussparung auf, deren Längsränder von umgebogenen Abschnitten des ersten Kanülenabschitts 10 erfasst werden, so dass ein Fixieren des zungen- oder blattförmigen Schutzelementes 30 bei gleichzeitiger Relativverschiebung zu dem ersten Kanülenabschnitt 10 ermöglicht wird. Hierzu ist der Schlitz in dem zungenförmigen Schutzelement 30 länger als die aus dem ersten Kanülenabschnitt 10 herausgebogenen Abschnitte, die in Fig. 6 mit den Bezugszeichen 36, 38 gekennzeichnet sind. Um die Abschnitte 36, 38 zu bilden, ist der erste Kanülenabschnitt 10 geschlitzt (Bezugszeichen 40), um sodann die Abschnitte 36, 38 umzubördeln und somit an den Längsrändern des Längsschlitzes 42 des zungenförmigen Schutzelementes anzuliegen. Es ergibt sich folglich ein Ausschnitt 39 in dem ersten Kanülenabschnitt 40, der von den Abschnitten 36, 38 begrenzt ist, wie die Fig. 8 verdeutlicht. In der Fig. 8 ist das Schutzelement 30 nicht eingezeichnet.

Von den Seitenrändern des Schutzelementes 30 gehen flügelartige Abschnitte 44, 46 aus, auf deren Längsrändern 45, 47 abschnittsweise die Längsränder 48, 50 des zweiten Kanülenabschnitts 12 aufliegen. Hierdurch ist sichergestellt, dass der zweite Kanülenabschnitt 12 nicht unkontrolliert in das Innere des ersten Kanülenabschnitts 10 hineingedrückt werden kann. Des Weiteren ergibt sich aus der Fig. 5, dass der zweite Kanülenabschnitt 12 im Bereich der flügelartigen Abschnitte 44, 46 jeweils eine Stufe 40 aufweist, die an den flügelartigen Abschnitten 44, 46 anliegt. Dies wird durch die zeichnerische Darstellung der Fig. 5 deutlich, ohne dass es näherer Erläuterungen bedarf.

Erfolgt nun eine Längsverschiebung des ersten Kanülenabschnitts 10 zu dem zweiten Kanülenabschnitt 12, so wird aufgrund des Anliegens der Stufen 60 des zweiten Kanülenabschnitts 12 an den flügelartigen Abschnitten 44, 46 das Schutzelement 30 während des Verstellens des ersten Kanülenabschnitts 10 über die Punktionsspitze 32 geschoben. Die Länge des Schlitzes 42 und der flügelartigen Abschnitte 36, 38 sowie Hebel 20 und Abstützung 26 sind so aufeinander abgestimmt, dass sodann ein Abziehen des ersten Kanülenabschnitts 10 von dem zweiten Kanülenabschnitt 12 erfolgt, wobei der distal abgewinkelte Abschnitt 34 des Schutzelementes 30 die Spitze 32 abdeckt, wie dies die Fig. 5 verdeutlicht.

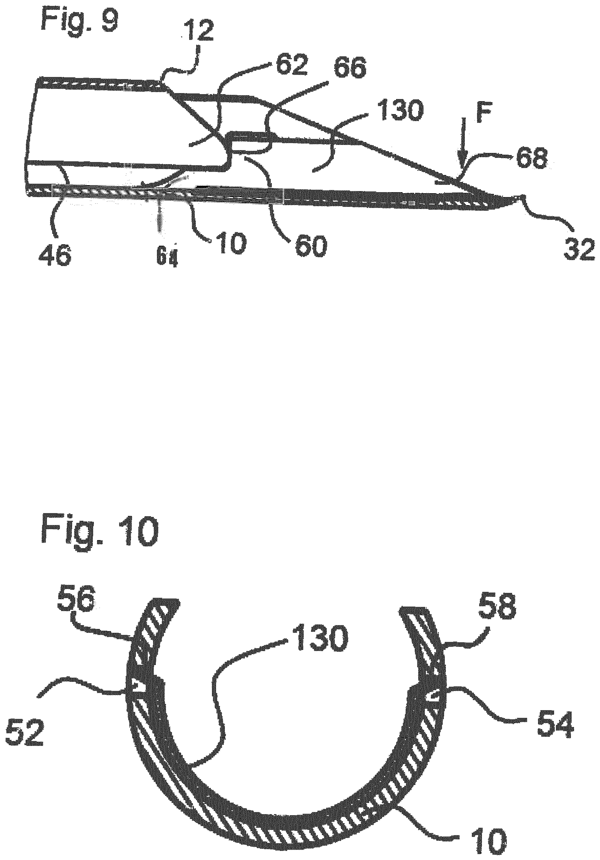

Den Fig. 9 bis 11 ist eine weitere Ausführungsform einer dem prinzipiellen Aufbau der anhand der Fig. 1 bis 4 beschriebenen Kanüle zu entnehmen, die jedoch in Bezug auf das Schutzelement von dem der Fig. 5 bis 8 abweicht. Ist das Schutzelement 30 als zungen- oder schalenförmig bezeichnet worden, so zeigt die Schnittdarstellung gemäß Fig. 10, dass nach der zweiten Ausführungsform ein Schutzelement 130 vorzugsweise über einen größeren Bogen an der Innenfläche des ersten Kanülenabschnitts 10 anliegt als das Schutzelement 30. Zum Fixieren des Schutzelementes 130 weist der erste Kanülenabschnitt 10 in Längsrichtung verlaufende Langlöcher 52, 54 auf, in die seitlich abragende Vorsprünge 56, 58 des Schutzelementes 130 eingreifen, wodurch eine Relativverschiebung zwischen dem Schutzelement 130 und dem ersten Kanülenabschnitt 10 in nachstehender Art ermöglicht wird.

Wie die Fig. 9 verdeutlicht, weist das Schutzelement 130 in seinem jeweiligen Längsseitenrandbereich eine Stufe 60 auf, gegen den die distalen Längsrandabschnitte 62 des zweiten Kanülenabschnitts 12 zum Anliegen kommen. Die Stufe 60 wird von einem parallel zur Längsachse des zweiten Kanülenabschnitts 12 verlaufenden Abschnitt 64 proximal begrenzt, auf dem der zweite Kanülenabschnitt 12 mit seinem jeweiligen Längsrand 44, 46 aufliegt. Durch diese Konstruktion ist sichergestellt, dass der zweite Kanülenabschnitt 12 nicht im unerwünschten Umfang in den ersten Kanülenabschnitt 10 hineingedrückt werden kann. Des Weiteren weist das Schutzelement 130 im proximalen Bereich einen zungenförmigen Abschnitt 66 auf, durch den das Schutzelement 130 eine Krafteinlenkung in Richtung des distalen Bereichs 68 erfährt, wie durch den Pfeil F angedeutet wird. Durch das Zungenelement 66 wird quasi das Schutzelement 130 um die als Drehachsen wirkenden Vorsprünge 56, 58 in Richtung der Punktionsspitze 32 gekippt. Eine diesbezügliche Konstruktion ist dann zu wählen, wenn das Schutzelement 130 aus Metall besteht. Ist das Schutzelement 130 demgegenüber ein Kunststoffelement, so verrastet dieses sodann mit der Punktionsspitze 32, wenn das Schutzelement 130 mit seinem distalen Ende die Punktionsspitze 32 überstreicht. Dies erfolgt entsprechend den zuvor erfolgten Erläuterungen durch die axiale Relativbewegung zwischen dem ersten Kanülenabschnitt 10 und dem zweiten Kanülenabschnitt 12 bei Betätigen des Hebels 20 in Richtung des Pfeils 24, also bei einer Krafteinleitung in Richtung distalen Bereichs der Kanüle. So wird aufgrund des Anliegens des distalen Bereichs 62 des zweiten Kanülenabschnitts 12 an den Stufen 60 des Schutzelementes 130 beim Zurückziehen des ersten Kanülenabschnitts 10 das Schutzelement 130 an einem Mitbewegen gehindert, so dass das Schutzelement 130 über die Punktionsspitze 32 geschoben wird und mit dieser verrasten kann. Alternativ oder ergänzend kann der distale Bereich des Schutzelementes 130 abgewinkelt werden, wie dies im Zusammenhang mit der Fig. 5 verdeutlicht worden ist.

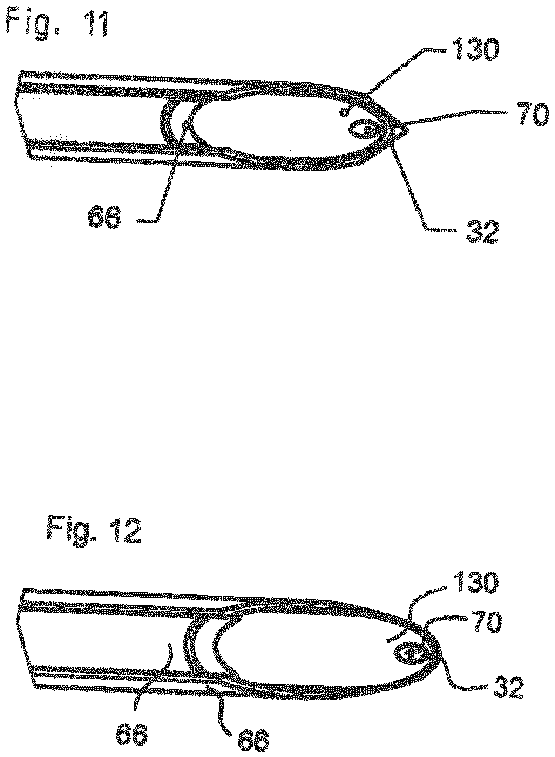

Das Zungen- oder Federelement 66 ist erwähntermaßen dann erforderlich, wenn das Schutzelement 130 aus einem Blechmaterial besteht. Um ungeachtet der Krafteinleitung in den distalen Bereich sicherzugehen, dass sich das Schutzelement 130 sodann nicht von der Spitze 32 lösen kann, sollte das Schutzelement 130 in seinem distalen Bereich eine Aussparung 70 aufweisen, in die die Spitze 32 einrasten kann, so dass ein unkontrolliertes Lösen des Schutzelements 130 von der Spitze 32 ausgeschlossen ist. Dies soll prinzipiell anhand der Fig. 12 verdeutlicht werden. Man erkennt, dass die Schutzkappe derart zu dem ersten Kanülenabschnitt 10 verstellt ist, dass die Öffnung 70 von der Punktionsspitze 32 durchsetzt ist, dass also ein Verrasten des Schutzelementes 130 erfolgt.

Als besonders vorteilhaft hat sich gezeigt, wenn der erste oder äußere Kanülenabschnitt 10, der den zweiten oder inneren Kanülenabschnitt 12 abschnittsweise umschlingt, wobei der Umschlingungswinkel von proximal in Richtung des distalen Bereichs, also in Richtung des Schutzelementes 30, 130 zunimmt, wobei im proximalen Bereich der Umschlingungswinkel kleiner als 180° sein kann, also im Schnitt das Bogenmaß kleiner als π ist. Ungeachtet dessen ist aufgrund der Spannung im distalen Bereich, in dem der erste Abschnitt 10 den zweiten Abschnitt 12 vorzugsweise mit einem Winkel von 245° bis 310° umschlingt, sichergestellt, dass ein unkontrolliertes Lösen des ersten und zweiten Kanülenabschnitts 10, 12 voneinander nicht erfolgt, vielmehr ein Abziehen allein durch die erfindungsgemäße konstruktive Lösung möglich ist.

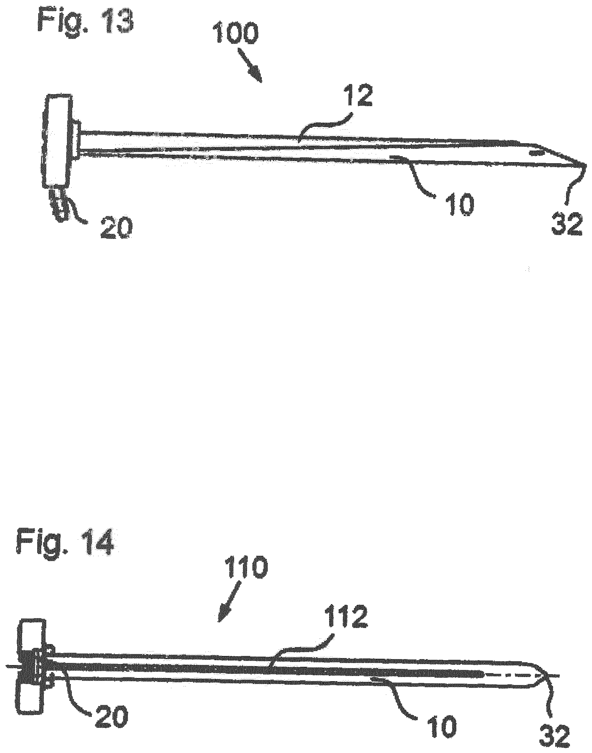

Dies soll rein prinzipiell anhand der Fig. 13 verdeutlicht werden. Dieser ist in Seitenansicht eine Kanüle 100 mit dem ersten oder äußeren Kanülenabschnitt 10 und dem inneren oder zweiten Kanülenabschnitt 12 zu entnehmen, wobei der erste Kanülenabschnitt 10 den zweiten Kanülenabschnitt 12 im distalen Bereich, also im Bereich der Spitze 32 stärker umschlingt als im proximalen Bereich, also im Bereich der Mechanik, mittels der der erste und zweite Kanülenabschnitt 10, 12 axial zueinander verschoben und sodann der erste Kanülenabschnitt 10 von dem zweiten Kanülenabschnitt 12 radial abgezogen wird. Im distalen Bereich kann der Umschlingungswinkel im Bereich zwischen 240° und 310° liegen, wohingegen im proximalen Bereich der Umschlingungswinkel im Bereich zwischen 180° und 120° liegen kann, um nur beispielhaft Zahlen zu nennen. Aus der Prinzipdarstellung der Fig. 13 erkennt man des Weiteren den Griff, der die Funktion des Abstützelementes 16 für den Hebel 20 ausübt. Zu erwähnen ist, dass in Fig. 13 die Änderung des Umschlingungswinkels übertrieben dargestellt ist.

Um das Abziehen zu erleichtern, kann des Weiteren vorgesehen sein, dass der erste Kanülenabschnitt 10 in dem der Öffnung gegenüberliegenden Bereich materialgeschwächt ist. Dabei sollte im Bereich der Spitze 36 eine solche Materialschwächung nicht vorgesehen sein. Diese sollte bevorzugt im Abstand von 2 cm bis 3 cm zu der Spitze 32 beginnen. Diese Materialschwächung bewirkt, dass das Abziehen des ersten Kanülenabschnitts 10 von dem zweiten Kanülenabschnitt 12 erleichtert wird.

Eine entsprechende Materialschwächung soll bei einer Kanüle 110 entsprechend der Fig. 14 rein prinzipiell verdeutlicht werden. Aus der Rückansicht in der Fig. 14 wird erkennbar, dass der erste Kanülenabschnitt 10 diametral gegenüberliegend zu seinem Längsschlitz materialgeschwächt ist, wobei die Schwächung beabstandet zur Kanülen- bzw. Punktionsspitze 32 beginnt.

Dieses Lösen des ersten Kanülenabschnitts 10 von dem zweiten Kanülenabschnitt 12 kann ergänzend dadurch erleichtert werden, dass vom proximalen Bereich ausgehend von der der Öffnung des ersten Kanülenabschnitts 10 gegenüberliegenden Seite, also im Bereich des Hebels 20, ein Längsschlitz eingebracht wird, der eine Länge von 2 cm bis 3 cm aufweisen kann. Benachbart zum Schlitz ist sodann der erste Kanülenabschnitt 10 abgewinkelt, weist also rechtwinklig abgebogene Laschen auf, um mit diesen jeweils ein Betätigungselement wie Hebel zu verbinden, d. h, jede Lasche weist einen entsprechenden Hebel auf. Ist die erforderliche Längsrelativbewegung zwischen erstem und zweiten Kanülenabschnitt 10, 12 erfolgt, so dass das Schutzelement 30, 130 die Spitze 32 abdeckt, so können die Griffe aufeinanderzu bewegt werden, wodurch das Abziehen erleichtert wird.

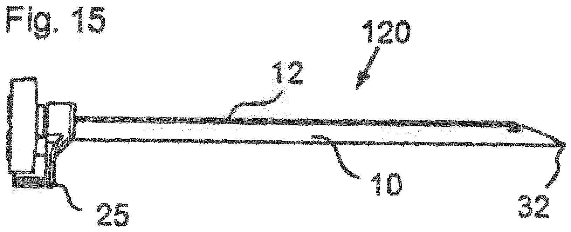

Eine einen proximalen Schlitz aufweisende Kanüle 120 ist der Fig. 15 zu entnehmen. In der zeichnerischen Darstellung der Fig. 15, die eine Seitenansicht der Kanüle 120 wiedergibt, ist erkennbar, dass der erste oder äußere Kanülenabschnitt 10 den inneren oder zweiten Kanülenabschnitt 12 mit gleichbleibendem Umschlingungswinkel aufnimmt, ohne dass hierdurch eine Beschränkung der Erfindung erfolgt.

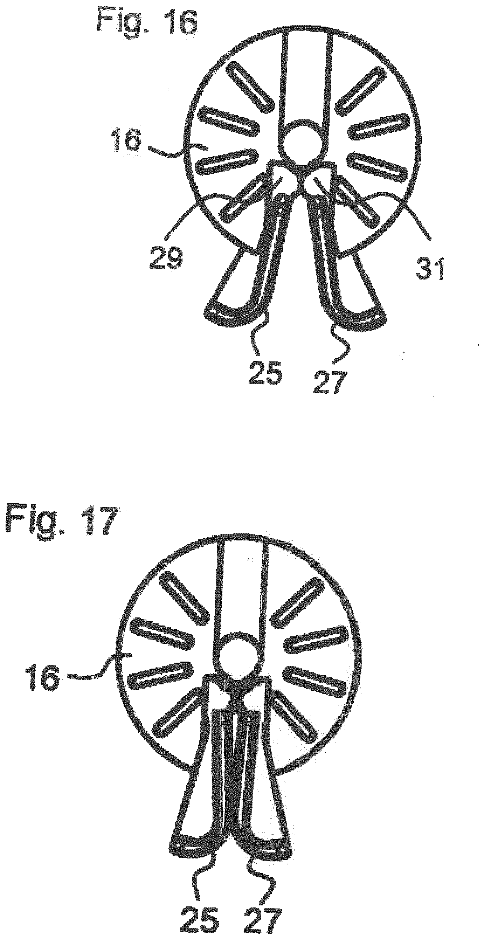

Wird eine Kanüle mit einem entsprechenden proximal verlaufenden Schlitz benutzt, so ergibt sich bezüglich des Betätigungselementes eine Konstruktion, wie diese prinzipiell den Fig. 16 und 17 zu entnehmen ist. Von dem zweiten Kanülenabschnitt 12 geht ein als Abstützelement dienender Griff aus, der eine Konstruktion wie die der Fig. 3 und 4 aufweisen kann. An dem Abstützelement sind aufgrund der zwei durch den Schlitz getrennten Abschnitte des ersten Kanülenabschnitts 10 zwei Hebelelemente 25, 27 in zuvor beschriebener Weise verriegel- und abstützbar, wobei die Hebelelemente 25, 27 in Nutzstellung der Kanüle entsprechend der Darstellung gemäß Fig. 16 zueinander beabstandet sind. Auf die Hebelelemente 25, 27 muss zum axialen Verschieben des ersten zu dem zweiten Kanülenabschnitt 10, 12 in Richtung des Pfeils 24 eine Kraft einwirken. Soll das Abziehen des äußeren Kanülenabschnitts 10 von dem zweiten Kanülenabschnitt 12 erfolgen, so werden die Hebelelemente 25, 27 zusammengedrückt (Fig. 17), wodurch ein Aufweiten des ersten Kanülenabschnitts 10 entsprechend der Breite des Schlitzes erfolgt. Hierdurch bedingt wird das Abziehen des ersten Kanülenabschnitts 10 von dem zweiten Kanülenabschnitt 12 erleichtert. Um die Hebelelemente 25, 27 zusammenzudrücken, ist beispielhaft vorgesehen, dass die Hebelelemente 25, 27 über Vorsprünge 29, 31 abstützbar und drehbar sind.

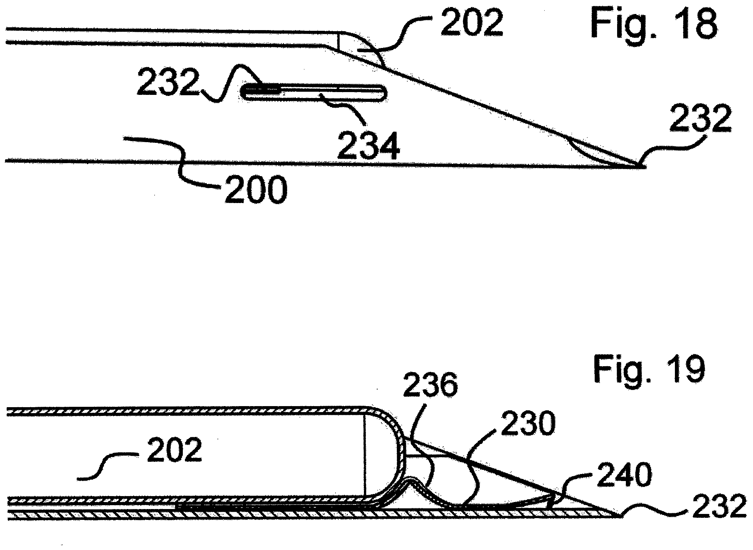

Anhand der Fig. 18 bis 35 sollen weitere Ausführungsformen von Schutzelementen erläutert werden, mittels derer die Spitze einer Kanüle derart gesichert wird, dass die Verletzungsgefahr für einen Nutzer oder einen Patienten ausgeschlossen bzw. minimiert wird. Als Kanülen - nicht zum Gegenstand der Erfindung gehörend-werden abweichend von den Ausführungsbeispielen der Fig. 1 bis 7 solche verwendet, die nicht zweischalig ausgebildet sind, also nicht einen ersten und einen zweiten Kanülenabschnitt aufweisen, die ineinandergreifen, sondern aus einem längsgeschnittenen Hülsenkörper bestehen, also sogenannte Rinnenkanülen sind, die gleichfalls insbesondere für suprapubische Punktion benutzt werden.

Entsprechende Kanülen sind über ihre Länge geschlitzt, d. h., sie bilden eine Kanülenschale, die um einen Winkel von 200° bis 270° reicht. Der Hülsenkörper bzw. die Schale weisen dabei im Schnitt grundsätzlich auch die Geometrie eines Abschnitts eines Kreisbogens auf.

Die nachstehend beschriebenen Ausführungsbeispiele von Schutzelementen sind jedoch nicht auf entsprechende Rinnenkanülen beschränkt, sondern können auch bei Spaltkanülen zum Einsatz gelangen, die entsprechend dem Ausführungsbeispiel der Fig. 1 bis 17 aus zwei ineinandergreifenden im Schnitt kreisbogenförmigen Abschnitten bestehen. Eine Verwendung für Spaltkanülen, die im Ausgangszustand einteilig und umlaufend geschlossen sind und nach Einführen eines Katheters aufgetrennt werden, ist gleichfalls möglich.

In den Fig. 18 bis 21 ist der distale Abschnitt einer Kanüle 200 dargestellt, die entsprechend zuvor erfolgter Erläuterungen als längsgeschnittener Hülsenkörper ausgebildet sind. Im distalen Bereich weist die Kanüle 200 eine Punktionsspitze 232 auf. Von der Kanüle 200, d. h. dem Hülsenkörper wird ein Katheter 202 geführt aufgenommen, der in Längsrichtung der Kanüle 200 verschiebbar ist, um z. B. in einen Körperhohlraum eingeführt zu werden. Um nach dem Setzen des Katheters 202 die Kanüle 200 aus dem Körper herauszuziehen und sodann von dem Katheter 202 zu lösen, ohne dass eine Verletzungsgefahr für den Benutzer bzw. den Patienten erwächst, wird ein in Längsrichtung der Kanüle 200 verschiebbares Schutzelement 230 benutzt, das sich abschnittsweise entlang der Innenseite des Kanülenkörpers erstreckt und seitlich abragende Vorsprünge 232 aufweist, die in Längsschlitze 234 des Kanülenkörpers eingreifen. Hierdurch erfolgt eine axiale Führung des Schutzelementes 230. Ist in den Figuren nur ein Schlitz 234 dargestellt, so verläuft diametral gegenüberliegend ein entsprechender Schlitz, in den gleichfalls ein Abschnitt des Schutzelementes 230 eingreift.

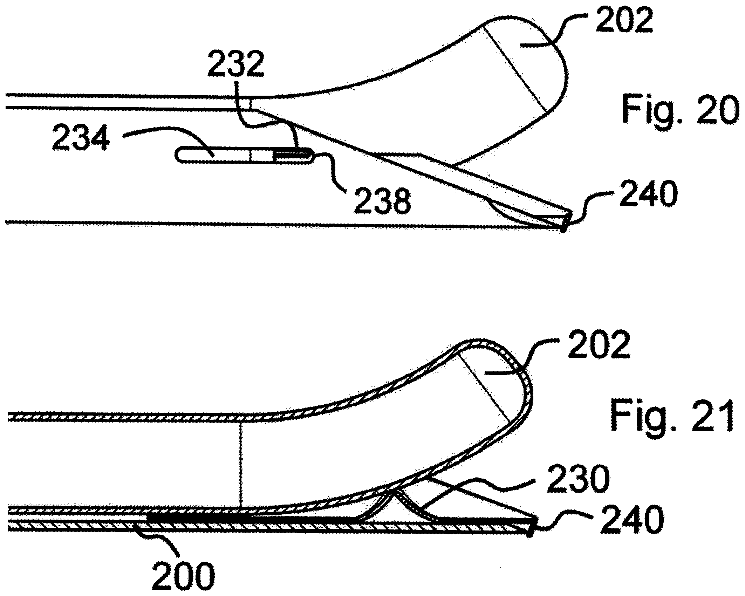

Wie die Schnittdarstellungen gemäß der Fig. 19 und 21 verdeutlichen, weist das Schutzelement 230 einen als Mitnehmer zu bezeichnenden und in das Kanüleninnere ragenden Vorsprung 236 auf, durch den das Schutzelement 230 dann in Richtung des distalen Endes der Kanüle 200, also der Punktionsspitze 232 mitgenommen und in Richtung Kanülenspitze geschoben wird, wenn der Katheter 202 an dem Vorsprung 236 zu liegen kommt. Das Schutzelement 230 wird solange von dem Katheter 202 mitgenommen, bis der Vorsprung 232 an der distalen Begrenzung 238 des Schlitzes 234 anliegt, wie die Fig. 20 verdeutlicht. In dieser Position kann ein hakenförmig gebogener Bereich 140 im distalen Ende des Schutzelementes 230 die Punktionsspitze 232 hintergreifen und mit dieser verrasten, wie sich aus den Fig. 20 und 21 ergibt. Das Schutzelement 230 ist somit durch Anliegen des Vorsprungs 232 an der Begrenzung des Schlitzes 234 und das Hintergreifen der Punktionsspitze 232 von dem hakenförmigen distalen Abschnitt 240 fixiert, so dass eine Längsverschiebbarkeit nicht mehr möglich ist. Somit ist die Punktionsspitze 232 bleibend durch das Schutzelement 230, d. h. dessen distalen Bereich abgedeckt mit der Folge, dass beim Herausziehen der Kanüle 200 und dem Lösen von dem Katheter 202 eine Verletzungsgefahr ausgeschlossen ist.

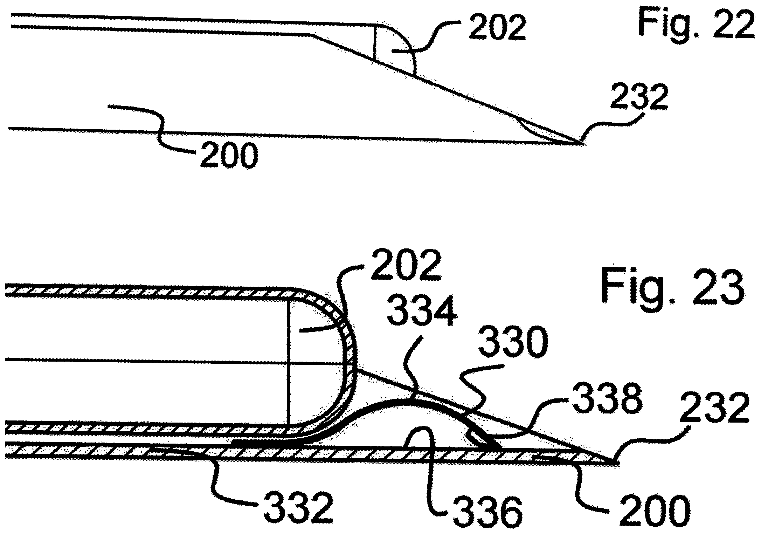

Eine vierte Ausführungsform eines Schutzelementes 330 ist den Fig. 22 bis 27 zu entnehmen. Dabei wird das Schutzelement 330 erneut anhand einer Kanüle erläutert, die aus einem längsgeschnittenen hülsenförmigen Körper besteht, so dass gleiche Bezugszeichen wie im Zusammenhang mit den Fig. 18 bis 21 verwendet werden.

Abweichend von dem Ausführungsbeispiel der Fig. 18 bis 21 ist das Schutzelement 330 nicht insgesamt längsverschiebbar innerhalb der Kanüle 200 verschiebbar, vielmehr in einem proximalen Bereich 332 mit der Kanüle 200 fest verbunden, wie sich prinzipiell aus der Fig. 23 ergeben soll. Das Verbinden zwischen dem Schutzelement 330 und der Kanüle 200 kann durch Schweißen, Kleben, Nieten oder auf andere geeignete Weise erfolgen, wobei medizinische Belange zu berücksichtigen sind.

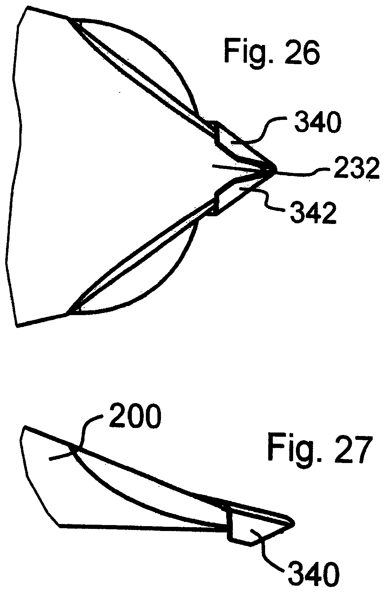

Im nicht aktivierten Zustand weist das Schutzelement 330 einen in Richtung der Mittelachse der Kanüle 200 gebogenen vorgespannten Abschnitt 334 auf, wobei die Höhe des bogenförmigen Abschnitts 334 derart ist, dass beim Verschieben des Katheters 202 dieser erfasst und in Richtung Innenseite 336 der Kanüle 200 gedrückt wird. Hierdurch erfolgt eine scheinbare Verlängerung des Schutzelementes 330 derart, dass sein distales Ende 338 entlang der Innenseite 336 der Kanüle 200 in Richtung der Punktionsspitze 232 verschoben wird und dann, wenn der Katheter 202 den bogenförmigen Abschnitt 334 fast vollständig auf die Innenseite 336 gedrückt hat, der distale Bereich 338 die Punktionsspitze 232 überragt und diese aufnimmt, wie sich aus den Fig. 26 und 27 ergibt. Hierzu ist das in Draufsicht streifen- oder zungenförmige Schutzelement 330 in seinem distalen Bereich 338 derart gebildet, dass sich in Draufsicht eine Dreiecksgeometrie ergibt, wobei die Ränder 340, 342 nach außen umgebogen sind und somit eine taschenförmige Aufnahme für die Punktionsspitze 332 bilden, wie sich selbsterklärend aus den Fig. 26 und 27 ergibt.

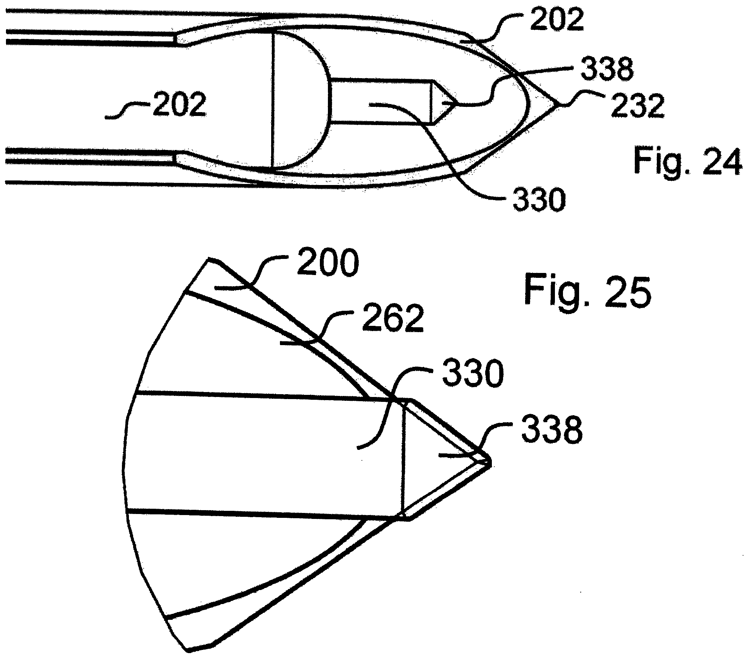

Die Ausgangslage, die der Fig. 23 entspricht, ist noch einmal in Fig. 24 in Draufsicht auf die Kanüle 200 dargestellt. In der Fig. 25 ist von der Unterseite das Schutzelement 330 in aktiver Position dargestellt. Der Katheter 202 ist nicht gezeichnet.

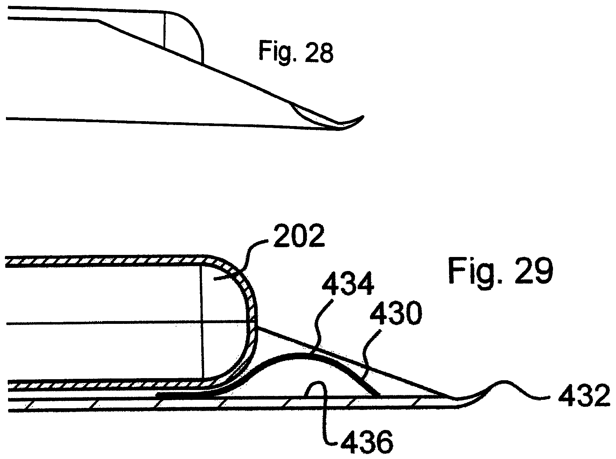

Eine besonders hervorzuhebende Ausführungsform einer fünften Ausführungsform eines Schutzelementes 430 ist den Fig. 28 bis 33 zu entnehmen, wobei das Schutzelement 430 in Bezug auf die Befestigung und den Verlauf dem Schutzelement 330 entspricht. Mit anderen Worten ist das Schutzelement 430 in seinem proximalen Bereich 432 mit der Innenseite 436 einer im Ausführungsbeispiel ebenfalls einschaligen Kanüle 400 verbunden, die sich von der Kanüle 200 dahingehend unterscheidet, dass die Punktionsspitze 432 nach innen gebogen ist, wie die zeichnerischen Darstellungen verdeutlichen.

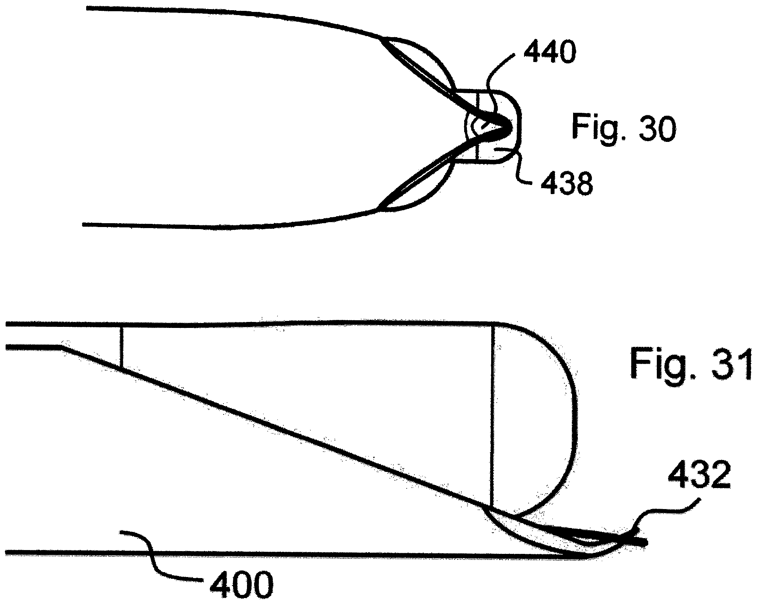

Um die Punktionsspitze 432 zu sichern, weist das im nicht aktivierten Zustand gleichfalls einen bogenförmig verlaufenden Abschnitt aufweisende Schutzelement 430 in seinem distalen Bereich 438 eine Öffnung 440 auf, die bei aktiviertem Schutzelement 430 von der Punktionsspitze 432 durchsetzt wird, wie sich insbesondere aus den Fig. 31, 32 und 33 ergibt.

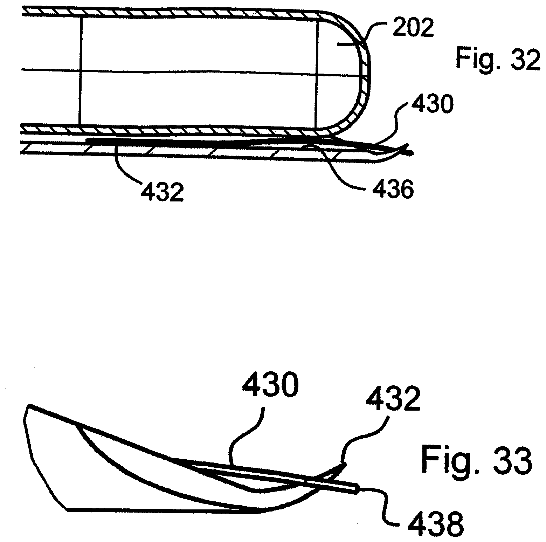

Im Ausgangszustand, wenn das Schutzelement 430 noch nicht aktiviert ist, verläuft der bogenförmige Abschnitt 434 vor dem distalen Bereich des Katheters 202, wie die Schnittdarstellung der Fig. 29 verdeutlicht. Eine Seitenansicht der Anordnung ist der Fig. 28 zu entnehmen. Im aktivierten Zustand, also in dem Zustand, in dem der Katheter 202 den bogenförmigen Abschnitt 434 im erforderlichen Umfang in Richtung der Innenfläche 436 der Kanüle 400 gedrückt hat, damit die Punktionsspitze 432 das Loch 440 im distalen Bereich 430 des Schutzelementes durchsetzt, ist den Fig. 31 und 32 zu entnehmen. Eine vergrößerte Darstellung der Punktionsspitze 432 mit dem diese abdeckenden distalen Bereich 438 des Schutzelementes 430 ergibt sich aus der Fig. 33. Anhand dieser Darstellung soll auch der Begriff Abdecken verdeutlicht werden. Abdecken bedeutet nicht zwingend, dass die Punktionsspitze 432 unmittelbar abgedeckt ist. Vielmehr ist der Bereich der Punktionsspitze 432 von dem Schutzelement 430, d. h. dessen distalen Bereich 430 in einem Umfang umgeben, dass bei einer normalen Handhabung der Kanüle 400 eine Berührung mit der Kanülenspitze 432 an und für sich nicht erfolgen kann.

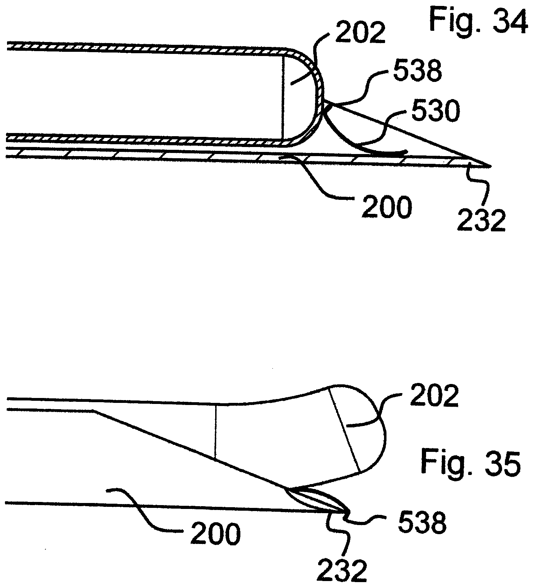

Eine sechste Ausführungsform eines Schutzelementes 530 ergibt sich aus den Fig. 34 und 35. Anhand dieser Figuren soll rein prinzipiell verdeutlicht werden, dass auch ein verschwenkbares Schutzelement, wie das Schutzelement 530, die Funktion ausüben kann, eine Kanülenspitze, d. h. die Punktionsspitze 232 abzudecken. So kann das Schutzelement 530 mittels eines nicht dargestellten Verbindungs- wie Scharnierelements, über das das Schutzelement 530 mit der Kanüle 200 verbunden ist, geschwenkt werden und zwar dann, wenn der Katheter 202 in Richtung der Punktionsspitze 232 verschoben wird, wie sich aus einem Vergleich der Fig. 34 und 35 ergibt. Dabei kann das Schutzelement 530 derart vorgespannt sein, dass dann, wenn das Schutzelement 530 mit seinem distalen Ende 538 die Punktionsspitze 232 abdeckt, in dieser Position verbleibt.

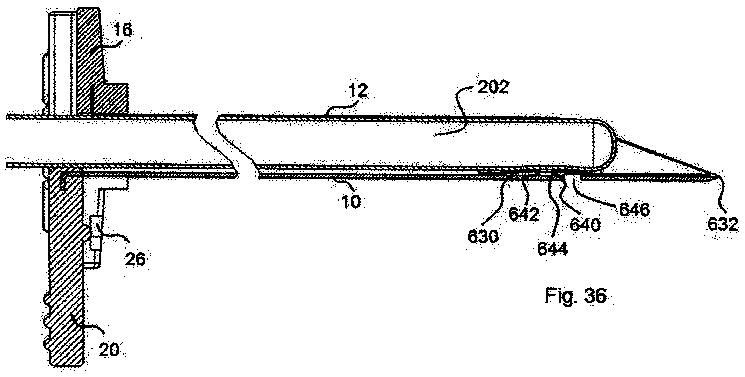

Weitere Ausführungsformen von Schutzelementen, mittels derer eine Spitze einer Kanüle so gesichert wird, dass einer Verletzung durch Nutzer oder Patienten dem Grunde nach ausgeschlossen ist, sind den Fig. 36 bis 42 zu entnehmen. Dabei werden die Schutzelemente im Zusammenhang mit Kanülen verwendet, die zweischalig ausgebildet sind, also einen konstruktiven Aufbau aufweisen, wie dieser anhand der Fig. 1 bis 17 beschrieben worden ist. Auf die diesbezüglichen Ausführungen wird ausdrücklich Bezug genommen, so dass auch für gleiche Elemente grundsätzlich gleiche Bezugszeichen verwendet werden, um Wiederholungen zu vermeiden.

Ungeachtet dessen ist jedoch auch die Möglichkeit gegeben, dass die entsprechenden Schutzelemente in einschaligen Kanülen - nicht zum Gegenstand der Erfindung gehörend - eingesetzt werden.

Den Fig. 36 bis 38 ist eine Kanüle 600 zu entnehmen, die entsprechend der Fig. 1 und 2 aus einem ersten und zweiten Abschnitt 10, 12 besteht, wobei der erste und der zweite Kanülenabschnitt 10, 12 im Schnitt eine Kreisbogen-Geometrie aufweisen kann. Ganz allgemein kann jedoch von einem längsgeschnittenen Hülsenkörper gesprochen werden, wobei Bogenlänge des ersten oder äußeren Kanülenabschnitts 10 und die des zweiten oder inneren Kanülenabschnitts 12 derart ausgelegt sind, dass entsprechend der erfindungsgemäßen Lehre ein Abziehen erfolgen kann, d. h., dass die Abschnitte zueinander radial, also quer zur Längsachse der Kanüle 600 auseinandergezogen werden, so dass ein Trennen voneinander erfolgt und somit eine von der Kanüle 600 durchsetzter Katheter freiliegt. Der Katheter ist entsprechend der Ausführungsbeispiele der Fig. 18 bis 35 mit dem Bezugszeichen 202 gekennzeichnet.

Entsprechend der Fig. 1 bis 17 ist der erste Kanülenabschnitt 10 mit dem Hebel 20 verbunden, der sich auf den Abschnitt 26 des Griffs 16 abstützt, der mit dem inneren oder zweiten Kanülenabschnitt 12 verbunden ist.

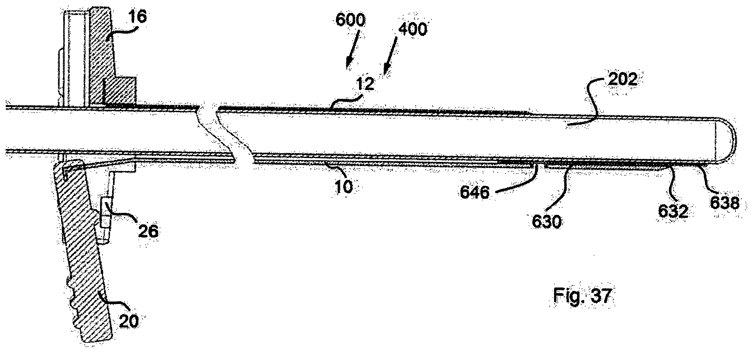

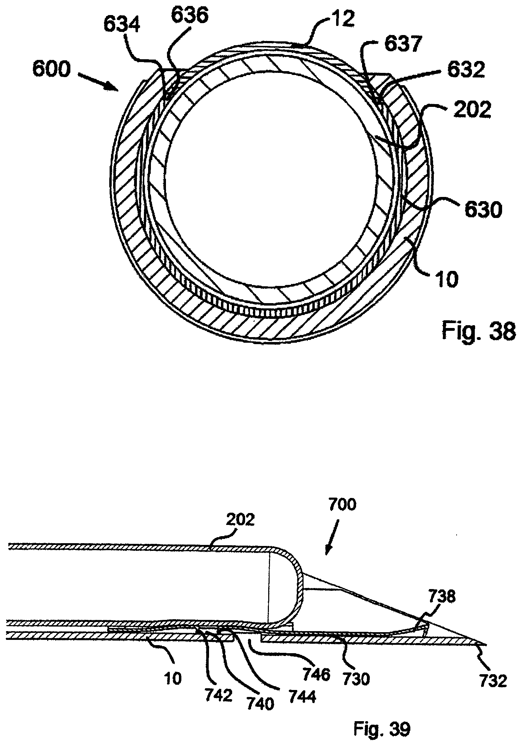

In Fig. 36 ist die Kanüle 600 bei sich in dieser befindlichem Katheter 202 und einem nicht aktivierten Schutzelement 630 dargestellt. Das Schutzelement 630 ist im Ausführungsbeispiel der Fig. 36 bis 38 als schalenförmiger Körper ausgebildet, der zumindest abschnittsweise an der Innenwandung des ersten Kanülenabschnitts 10 vorgespannt anliegt, so dass hierdurch eine Fixierung gegeben ist, sofern keine Längskräfte auf das Schutzelement 630 eingreifen. Gleichzeitig ergibt sich der Vorteil, dass durch das Schutzelement 630 der Innenquerschnitt des ersten Kanülenabschnitts 10 nicht merklich verkleinert wird, so dass nach wie vor ein problemloses axiales Verstellen des Katheters 220 möglich ist. Das Schutzelement 630 wird in einem Umfang in Richtung der Punktionsspitze 632 verschoben, dass diese vom distalen Bereich 638 des Schutzelements 630 abgedeckt ist, wie sich aus der Darstellung der Fig. 37 ergibt. Um das axiale Verschieben zu begrenzen, verrastet das Schutzelement Das Schutzelement 630 weist zumindest in seinem proximalen Bereich, vorzugsweise zumindest über die Hälfte seiner Länge im Schnitt eine Kreisbogen-Geometrie auf, wobei die Bogenlänge mehr als π beträgt, wie sich auch aus der Schnittdarstellung gemäß Fig. 38 ergibt. Die Längsränder 632, 634 des Schutzelementes verlaufen parallel zur Längsachse der Kanüle 600. Um ein sicheres Führen in Längsrichtung der Kanüle 600 und eine Unverdrehbarkeit des Schutzelementes 630 zu erreichen, liegen an den Längsrändern 632, 634 des Schutzelementes 630 die Längsränder 636, 637 des inneren oder zweiten Kanülenabschnitts 12 an.

Des Weiteren weist das Schutzelement 630 beabstandet zu seinem distalen Ende einen in Richtung der Kanülenwandung verlaufenden Vorsprung 640 auf. Der Vorsprung 640 kann durch zwei Flügel 642, 644 gebildet werden. Hierzu werden in dem Schutzelement 630 entsprechende Einschnitte eingebracht, um sodann die Flügel 642, 644 nach außen zu biegen. Somit kann eine Technik benutzt werden, wie diese auch im Zusammenhang mit dem Fig. 7 und 8 erläutert worden ist, so dass auf die diesbezüglichen Ausführungen verwiesen wird.

Um die Punktionsspitze 632, die im Ausführungsbeispiel der Fig. 36 bis 38 nicht nach innen gebogen ist, zu schützen, erfolgt ein Verschieben des Schutzelenmets 630 in Richtung der Punktionsspitze 632 dann, wenn der Hebel 20 in der zeichnerischen Darstellung entgegen dem Uhrzeigersinn verschwenkt wird. Hierdurch bedingt wird der erste Kanülenabschnitt 10 in Bezug auf den zweiten Kanülenabschnitt 12 nach links axial verstellt, also scheinbar verkürzt. Gleichzeitig wird das Schutzelement 630 entlang der Innenseite des ersten Kanülenabschnitts 10 in Richtung der Punktionsspitze 632 verschoben, da das Schutzelement 630 von dem zweiten Kanülenabschnitt fixiert ist. So ist insbesondere vorgesehen, dass der zweite Kanülenabschnitt 12 formschlüssig mit dem Schutzelement 630 zusammenwirkt.

Um ein axiales Verstellen in Richtung der Punktionsspitze 632 zu begrenzen, befindet sich in der Wandung des ersten Kanülenabschnitts 10 eine Öffnung 646, die sich im Verschiebeweg des Vorsprungs 640 befindet, so dass der Vorsprung 640 in die Öffnung 646 einrasten kann, wie ein Vergleich mit der Fig. 37 verdeutlicht. Insbesondere befindet sich die Öffnung 646 diametral zum Längsschlitz des ersten Kanülenabschnitts 10, der erwähntermaßen auch als längsgeschlitzter Hülsenkörper oder längsgeschlitze Hülse zu bezeichnen ist.

Um ein Mitnehmen des Schutzelementes 630 beim axialen Verstellen des ersten Kanülenabschnitts 610 zu dem zweiten Kanülenabschnitt 612 völlig auszuschließen, kann entsprechend den Erläuterungen im Zusammenhang mit der Fig. 9 in einen Ausschnitt des im Schnitt bogenförmigen Schutzelementes 630 eine Stufe des zweiten Kanülenabschnitts 612 eingreifen, ohne dass dies zeichnerisch dargestellt ist.

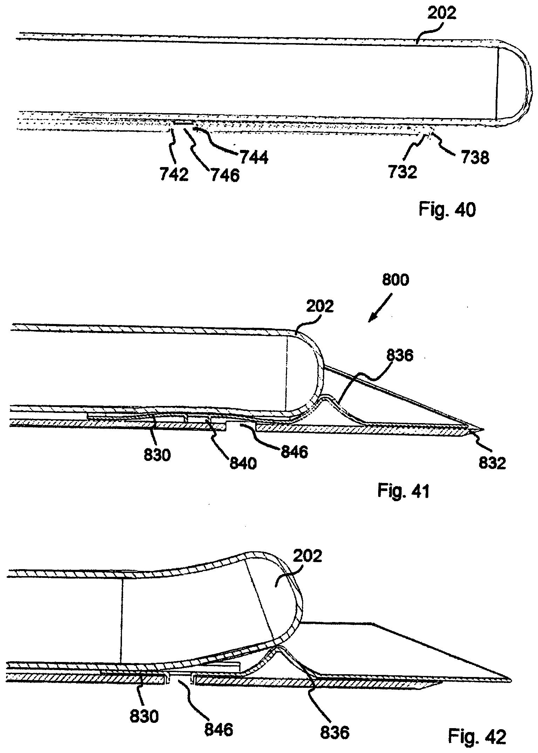

Eine weitere Ausführungsform eines Schutzelementes 730 ist den Fig. 39 und 40 zu entnehmen. Dabei unterscheidet sich das Schutzelement 730 von dem der Fig. 36 bis 38 einzig und allein in Bezug auf die Gestaltung des distalen Bereichs 738. Ansonsten weist das Schutzelement 730 gleichfalls einen über einen Bogen von mehr als 180° verlaufenden schalenförmigen Körper auf, der quasi federgespannt in dem ersten Kanülenabschnitt 710 fixiert ist und einen Vorsprung 740 mit Flügeln 742, 744 aufweist, die in die Durchgangsöffnungen 746 des ersten Kanülenabschnitts 10 bei aktiviertem Schutzelement 730 eingreifen, wie die Fig. 40 verdeutlicht.

Wird bei den Ausführungsbeispielen der Fig. 36 bis 40 das Schutzelement 630 bzw. 730 beim axialen Verstellen des ersten Kanülenabschnitts 10 zu dem zweiten Kanülenabschnitt 12 von dem zweiten Kanülenabschnitt 12 gehalten, um eine Längsverschiebung entlang des ersten Kanülenabschnitts 10 zum Abdecken der Kanülenspitze 632, 732 zu ermöglichen, so wird nach dem Ausführungsbeispiel einer den Fig. 41 und 42 zu entnehmenden Kanüle 800, deren Aufbau der der zuvor beschriebenen Kanüle 600, 700 und damit der Kanüle entsprechend den Fig. 1 bis 17 entspricht, ein Verschieben des Schutzelements 830 mittels des Katheters 202 ermöglicht, wie dies im Zusammenhang mit den Fig. 18 bis 21 erläutert worden ist, ohne dass jedoch das Schutzelement 830 in Schlitzen geführt werden muss, die in der Kanülenwandung vorhanden sind. Mit anderen Worten weist das Schutzelement 830 einen ins Innere der Kanüle 800 ragenden Vorsprung 836 auf, durch den das Schutzelement 830 in Richtung der Punktionsspitze 832 verstellt wird, wenn der Katheter 202 in dessen Richtung axial in der Kanüle 800 verstellt wird. Da entsprechend der zuvor erfolgten Erläuterungen das Schutzelement 830 einen durch umgebogene Ausschnitte gebildeten Vorsprung 640 aufweist, der selbstverständlich konstruktiv auch anders ausgebildet sein kann, ist ein weiteres axiales Verstellen des Schutzelementes 830 dann ausgeschlossen, wenn der Vorsprung 840 in die in der Kanüle 800 vorhandene Öffnung 846 eingreift und in dieser verrastet, wie dies der Fig. 42 zu entnehmen ist. Bei weiterem Verstellen des Katheters 202 wird dieser durch den Vorsprung 836 ausgelenkt, um in den gewünschten Bereich eines Körpers zu gelangen. In dieser Position darf der zweite Kanülenabschnitt 12 das Auslenken des Katheters 202 selbstverständlich nicht behindern. Im verrasteten Bereich des Schutzelements 836 befindet sich oberhalb des Vorsprungs 836 der zweite Kanülenabschnitt 812 nicht mehr.

Wie insbesondere ein Vergleich der Fig. 41 und 42 verdeutlicht, weist das Schutzelement 830 stirnseitig, also in seinem distalen Bereich, eine im Schnitt keilförmige Geometrie auf, die der der Kanüle 800 in ihrem distalen Bereich entspricht. Hierdurch wird zusätzlich sichergestellt, dass eine Verletzung am Stirnrand der Kanüle ausgeschlossen wird. Entsprechende Geometrien können auch die weiteren zuvor erläuterten Schutzelemente aufweisen.

Ist in den zeichnerischen Darstellungen erläutert worden, dass durch Verstellen des Hebels 20 der erste oder äußere Kanülenabschnitt 10 bzw. die äußere Schale axial zu dem zweiten oder inneren Kanülenabschnitt 12 oder der inneren Schale verstellt wird, so besteht selbstverständlich auch die Möglichkeit, dass von dem zweiten Kanülenabschnitt 12 ein Hebel ausgeht, der auf einem Abschnitt bzw. einem Griff des ersten Kanülenabschnitts abstützbar und zu diesem verschwenkbar ist. In diesem Fall würde der Hebel nicht in Richtung des distalen Endes der Kanüle verstellt werden, vielmehr von diesem weg.

Ist anhand der Fig. 36 bis 42 das jeweilige Schutzelement 630, 730, 830 im Zusammenhang mit einer zweischaligen Kanüle 600, 700, 800 beschrieben worden, so ist die Führung und das Verstellen des jeweiligen Schutzelementes auch dann bei einer einschaligen Kanüle - nicht zum Gegenstand der Erfindung gehörend-möglich, wenn das Schutzelement nicht nur in Richtung der Innenwandung der Schale vorgespannt ist, sondern zusätzliche eine axiale Führung gegeben ist. Diese kann dadurch realisiert werden, dass vom Bodens des schalenförmigen Körpers ein Vorsprung abragt, der in eine in axialer Richtung verlaufende Vertiefung des Schutzelementes eingreift oder umgekehrt.

## Patentansprüche

1. Kanüle (100, 110, 120, 200, 400, 600, 700, 800), insbesondere suprapubische Kanüle zum Einführen eines Katheters (202) in einen Körperhohlraum, mit einem ersten Kanülenabschnitt (10) in Form eines längsgeschnittenen ersten Hülsenkörpers, insbesondere mit im Schnitt einer Kreisbogen-Geometrie oder einer kreisbogenförmigen Geometrie, und einem beim Einführen eines Katheters mit diesem verbundenen zweiten Kanülenabschnitt (12) in Form eines längsgeschnittenen zweiten Hülsenkörpers, insbesondere mit im Schnitt einer Kreisbogen-Geometrie oder einer kreisbogenförmigen Geometrie, wobei einer der Kanülenabschnitte im distalen Ende eine Punktionsspitze (32, 632, 732, 832) aufweist, und wobei sich die miteinander verbundenen Kanülenabschnitte umschlingen, wobei der erste Kanülenabschnitt (10) einen Umschlingungswinkel in Bezug auf den zweiten Kanülenabschnitt (12) aufweist, der in einem senkrecht zur Längsachse des ersten Kanülenabschnitts verlaufenden Schnitt größer als 180° ist,
**dadurch gekennzeichnet,**
**dass** zum Lösen der Kanülenabschnitte (10, 12) voneinander einer der Kanülenabschnitte gegenüber dem anderen Kanülenabschnitt mittels zumindest eines von einem der Kanülenabschnitte ausgehenden und sich direkt oder indirekt an dem anderen Kanülenabschnitt abstützenden Betätigungselements (20, 25, 27) bei Krafteinwirkung auf das Betätigungselement quer zur Längsachse der Kanüle (100, 110, 120, 200, 400, 600, 700, 800) abziehbar ist, wobei das Betätigungselement ein Hebelelement ist, das über einen von diesem abragenden Vorsprung (22) an einem Abschnitt eines von dem anderen Kanülenabschnitt ausgehenden Abstützelements (16) abstützbar ist.

2. Kanüle nach Anspruch 1,
**dadurch gekennzeichnet.**
**dass** bei Krafteinwirkung des Betätigungselementes in Längsrichtung der Kanüle (100, 110, 120, 200, 400, 600, 700, 800), vorzugsweise in Richtung des distalen Bereichs der Kanüle, der Kanülenabschnitt (10), von dem das Betätigungselement ausgeht, zunächst in Längsrichtung der Kanüle entlang des anderen Kanülenabschnitts (12) verschiebbar ist, und dass sodann bei weiterer Krafteinwirkung der eine Kanülenabschnitt zumindest im proximalen Bereich von dem anderen Kanülenabschnitt lösbar ist.

3. Kanüle nach Anspruch 1 und Anspruch 2,
**dadurch gekennzeichnet,**
**dass** das Hebelelement (20, 25, 27) an einem rampenförmigen Abschnitt (26) des Abstützelements (16) abstützbar ist.

4. Kanüle nach zumindest einem der vorhergehenden Ansprüche,
**dadurch gekennzeichnet,**
**dass** das Abstützelement (16) ein sich quer zur Längsrichtung des zweiten Kanülenabschnitts (12) erstreckendes plattenförmiges Element ist, das in Richtung des Lösens der Kanülenabschnitte (10, 12) voneinander den ersten Kanülenabschnitt (10) mit einem Abstand umgibt, der ein Lösen der Kanülenabschnitte ermöglicht.

5. Kanüle nach Anspruch 1,
**dadurch gekennzeichnet,**
**dass** von dem ersten Kanülenabschnitt (10) ein entlang dessen Innenseite verstellbares Schutzelement (30, 130) ausgeht, das in Nutzposition der Kanüle beabstandet zu der Punktionsspitze (32) verläuft und nach Trennen der Kanülenabschnitte die Punktionsspitze abdeckt.

6. Kanüle nach Anspruch 5,
**dadurch gekennzeichnet,**
**dass** das Schutzelement (30, 130) bei der Längsverschiebung des ersten Kanülenabschnitts (10) zu dem zweiten Kanülenabschnitt (12) in Richtung der Punktionsspitze (32) durch Wechselwirken mit dem zweiten Kanülenabschnitt (12) verschiebbar ist.

7. Kanüle nach zumindest Anspruch 5 oder Anspruch 6,
**dadurch gekennzeichnet,**
**dass** das Schutzelement (30) ein zungen- oder rinnenförmiges Element ist, in dessen Längsrichtung ein Schlitz (42) verläuft, von dessen Längsrandseiten flügel artige Abschnitte (44, 46) ausgehen, dass von dem ersten Kanülenabschnitt (10) nach innen umgebogene und den Schlitz durchsetzende und an dessen Längsrandabschnitten anliegende Halteabschnitte (36, 38) ausgehen, die in Längsrichtung des ersten Kanülenabschnitts eine Länge aufweisen, die kürzer als Länge des Schlitzes ist, und dass in Nutzposition der Kanüle auf den Längsrändern (45, 47) der flügelartigen Abschnitte zumindest abschnittsweise Längsrandabschnitte (48, 50) des zweiten Kanülenabschnitts (12) aufliegen und dass der zweite Kanülenabschnitt in seinem distalen Bereich jeweils eine stufenartige Aussparung (60) in seinem Längsrandbereich aufweist, die an einer der flügelartigen Abschnitte zur Ermöglichung einer Relativbewegung des Schutzelementes zu dem ersten Kanülenabschnitt anliegt.

8. Kanüle nach zumindest einem der Ansprüche 5 bis 7,
**dadurch gekennzeichnet,**
**dass** der erste Kanülenabschnitt (10) in dessen Längsrichtung verlaufende Langlöcher (52, 54) aufweist, in die von dem Schutzelement (130) ausgehende Vorsprünge (56, 58) eingreifen, dass der zweite Kanülenabschnitt (12) mit dem Schutzelement derart wechselwirkt, dass bei Längsverschiebung des ersten Kanülenabschnitts zu dem zweiten Kanülenabschnitt das Schutzelement in Richtung der Punktionsspitze (32) und zum Abdecken dieser verschiebbar ist.

9. Kanüle nach zumindest einem der Ansprüche 5 bis 8,
**dadurch gekennzeichnet,**
**dass** das Schutzelement (130) proximal einen stufenförmigen Abschnitt (60) aufweist, an dem der distale Bereich des zweiten Kanülenabschnitts (12) bei Verschieben des ersten Kanülenabschnitts (10) zu dem zweiten Kanülenabschnitt anliegt, oder dass das Schutzelement (130) in seinem proximalen Bereich ein sich an Innenwandung des ersten Kanülenabschnitts (10) abstützendes Zungenelement (66) zur Krafteinleitung in den distalen Bereich des Schutzelements in Richtung der Innenseite des ersten Kanülenabschnitts aufweist, und/oder dass das Schutzelement (130) in seinem distalen Bereich eine Aussparung (70) aufweist, in die bei abgedeckter Punktionsspitze (32) die Punktionsspitze eingreift, und/oder das Schutzelement (30) distal abgewinkelt ist, wobei innerhalb des abgewinkelten Bereichs (34) die Punktionsspitze positionierbar ist.

10. Kanüle nach zumindest einem der Ansprüche 5 bis 9,
**dadurch gekennzeichnet,**
**dass** das Schutzelement (30, 130) zungen- oder rinnenförmig ausgebildet ist und außenseitig flächig oder im Wesentlichen flächig an Innenseite des ersten Kanülenabschnitts (10) anliegt, und/oder dass das Schutzelement (30, 130) aus zumindest einem Material aus der Gruppe Kunststoff, Metall besteht oder dieses enthält.

11. Kanüle nach zumindest einem der vorhergehenden Ansprüche,
**dadurch gekennzeichnet,**
**dass** der erste Kanülenabschnitt (10) senkrecht zur Längsachse zumindest abschnittsweise ein Bogenmaß B₁ mit B₁ > π und der zweite Kanülenabschnitt (12) ein Bogenmaß B₂ mit B₁ + B2 > 2 π aufweist, und /oder dass senkrecht zur Längsachse der Kanüle der erste Kanülenabschnitt (10) im proximalen Bereich ein Bogenmaß B₃ und der zweite Kanülenabschnitt (12) ein Bogenmaß B₄ mit B₃ < π und B₃ + B₄ > 2π aufweisen, und /oder dass der erste und der zweite Kanülenabschnitt im distalen Bereich, insbesondere bis in den Bereich des Schutzelementes (30, 130), jeweils ein Bogenmaß B₅ mit B₅ > π, insbesondere 1,2 π < B₅ ≤ 1,75 π aufweist.

12. Kanüle nach zumindest einem der vorhergehenden Ansprüche,
**dadurch gekennzeichnet,**
**dass** der erste Kanülenabschnitt (10) auf der zu seiner Längsöffnung gegenüberliegenden Seite durch z. B. Prägen materialgeschwächt ist.

13. Kanüle nach zumindest einem der vorhergehenden Ansprüche,
**dadurch gekennzeichnet,**
**dass** vom proximalen Querrand des ersten Kanülenabschnitts (10) auf der zu dessen Längsöffnung gegenüberliegender Seite ein Schlitz ausgeht und dass von jedem der zu dem Schlitz benachbarten Bereiche des ersten Kanülenabschnitts ein Betätigungselement (25, 27) ausgeht.

14. Kanüle nach zumindest einem der vorhergehenden Ansprüche,
**dadurch gekennzeichnet,**
**dass** zumindest der die Punktionsspitze (20) aufweisende erste Kanülenabschnitt (10) aus Metall besteht und dass ggfs. der erste und der zweite Kanülenabschnitt (10, 12) aus unterschiedlichen Materialien bestehen, wobei der zweite Kanülenabschnitt (12) aus Kunststoff besteht und vorzugsweise ein Spritzgussteil ist.

## Claims

1. Cannula (100, 110, 120, 200, 400, 600, 700, 800), in particular a suprapubic cannula for inserting a catheter (202) into a body cavity, with a first cannula section (10) in the form of a longitudinally split first sleeve member, in particular with an arc geometry or an arc-shaped geometry in section, and with a second cannula section (12) connected thereto during insertion of a catheter in the form of a longitudinally split second sleeve member, in particular with an arc geometry or an arc-shaped geometry in section, one of the cannula sections having at the distal end a punction tip (32, 632, 732, 832),
and where the cannula sections connected to one another wrap around, the first cannula section (10) having a wrap-around angle relative to the second cannula section (12) which is greater than 180° in a section vertical to the longitudinal axis of the first cannula section,
wherein
to release the cannula sections (10, 12) from one another one of said cannula sections is detachable from the other cannula section by means of at least one actuating element (20, 25, 27) extending from one of the cannula sections and directly or indirectly supported on the other cannula section when force is applied to the actuating element transversely to the longitudinal axis of the cannula (100, 110, 120, 200, 400, 600, 700, 800), said actuating element being a lever element which is supportable via a projection (22) protruding therefrom on a section of a supporting element (16) extending from the other cannula section.

2. Cannula according to claim 1,
**wherein**
when force is applied by the actuating element in the longitudinal direction of the cannula (100, 110, 120, 200, 400, 600, 700, 800), preferably in the direction of the distal area of the cannula, the cannula section (10) from which the actuating element extends is initially movable in the longitudinal direction of the cannula along the other cannula section (12), and when further force is applied the one cannula section is then releasable at least in the proximal area from the other cannula section.

3. Cannula according to claim 1 and claim 2,
**wherein**
the lever element (20, 25, 27) is supportable on a ramp-like section (26) of the supporting element (16).

4. Cannula according to at least one of the preceding claims,
**wherein**
the supporting element (16) is a plate-like element extending transversely to the longitudinal direction of the second cannula section (12) and which surrounds the first cannula section (10), in the direction of release of said cannula sections (10, 12) from one another, at a distance permitting release of said cannula sections.

5. Cannula according to claim 1,
**wherein**
from the first cannula section (10) extends a protective element (30,130) adjustable along its inside and which in the usage position of the cannula is at a distance from the punction tip (32) and covers said punction tip after separation of the cannula sections.

6. Cannula according to claim 5,
**wherein**
the protective element (30, 130) is movable during the longitudinal movement of the first cannula section (10) relative to the second cannula section (12) in the direction of the punction tip (32) by interaction with the second cannula section (12).

7. Cannula according to at least claim 5 and claim 6,
**wherein**
the protective element (30) is a tongue-shaped or trough-shaped element in whose longitudinal direction passes a slot (42) from whose longitudinal rim sides extend wing-like sections (44, 46), inward-bent retaining sections (36, 38) passing through the slot and in contact with its longitudinal rim sections extend from the first cannula section (10) and have in the longitudinal direction of the first cannula section a length shorter than the length of the slot, and in the usage position of the cannula, longitudinal rim sections (48, 50) of the second cannula section (12) rest on the longitudinal rims (45, 47) of at least some of the wing-like sections at least in sections, and the second cannula section has in its distal area a step-like recess (60) in its longitudinal rim area which is in contact with one of the wing-like sections to permit a movement of the protective element relative to the first cannula section.

8. Cannula according to at least one of claims 5 to 7,
**wherein**
the first cannula section (10) has slots (52, 54) running in its longitudinal direction in which engage projections (56, 58) extending from the protective element (130), and the second cannula section (12) interacts with the protective element such that during longitudinal movement of the first cannula section relative to the second cannula section the protective element is movable in the direction of the punction tip (32) and to cover the latter.

9. Cannula according to at least one of claims 5 to 8,
**wherein**
the protective element (130) has proximally a step-like section (60) with which the distal area of the second cannula section (12) is in contact during movement of the first cannula section (10) relative to the second cannula section, or the protective element (130) has in its proximal area a tongue element (66) supported on an inner wall of the first cannula section (10) for applying force into the distal area of the protective element in the direction of the inside of the first cannula section, and/or the protective element (130) has in its distal area a recess (70) into which engages the punction tip (32) when the latter is covered, and/or the protective element (30) is distally angled, the punction tip being positionable inside the angled area (34).

10. Cannula according to at least one of claims 5 to 9,
**wherein**
the protective element (30, 130) is designed tongue-shaped or trough-shaped and on the outside is in flat or substantially flat contact with the inside of the first cannula section (10), and/or the protective element (30, 130) consists of or contains at least one material from the group plastic, metal.

11. Cannula according to at least one of the preceding claims,
**wherein**
the first cannula section (10) vertical to the longitudinal axis has at least in some sections a radian B₁ where B₁ > π and the second cannula section (12) a radian B₂ where B₁ + B₂ > 2*π*, and/or
vertical to the longitudinal axis of the cannula the first cannula section (10) has in the proximal area a radian B₃ and the second cannula section (12) a radian B₄ where B₃ < π and B₃ + B₄ > 2π, and/or
the first and the second cannula sections in the distal area, in particular as far as the area of the protective element (30, 130), each have a radian B₅ where B₅ > *π,* in particular 1.2*π* < B₅ ≤ 1.75π.

12. Cannula according to at least one of the preceding claims,
**wherein**
the first cannula section (10) is materially weakened on the side opposite its longitudinal opening, for example by stamping.

13. Cannula according to at least one of the preceding claims,
**wherein**
a slot extends from the proximal transverse rim of the first cannula section (10) on the side opposite to its longitudinal opening, and an actuating element (25, 27) extends from each of those areas of the first cannula section adjacent to the slot.

14. Cannula according to at least one of the preceding claims,
**wherein**
at least the first cannula section (10) having the punction tip (20) consists of metal, and if desired the first and the second cannula sections (10, 12) consist of different materials, the second cannula section (12) consisting of plastic and being preferably an injection-moulded part.

## Revendications

1. Canule (100, 110, 120, 200, 400, 600, 700, 800), notamment canule suprapubienne pour l'introduction d'un cathéter (202) dans une cavité corporelle, avec une première section de canule (10) sous forme d'un premier corps de manchon fendu longitudinalement, notamment avec, en coupe, la géométrie d'un arc de cercle ou une géométrie en forme d'arc de cercle, et une seconde section de canule (12) reliée à celle-ci lors de l'introduction d'un cathéter, sous forme de second corps de manchon fendu longitudinalement, notamment avec, en coupe, la géométrie d'un arc de cercle ou une géométrie en forme d'arc de cercle, sachant qu'une des sections de canule présente une pointe de ponction (32, 632, 732, 832) sur l'extrémité distale,
et sachant que les sections de canule reliées entre elles s'entrelacent, sachant que la première section de canule (10) présente un angle d'enlacement par rapport à la seconde section de canule (12) qui, dans une coupe s'étendant perpendiculairement à l'axe longitudinal de la première section de canule, est supérieur à 180°,
**caractérisée en ce**
**que** pour détacher les sections de canule (10, 12) l'une de l'autre, une des sections de canule est extractible de l'autre section de canule au moyen d'au moins un élément d'actionnement (20, 25, 27) partant d'une des sections de canule et s'appuyant directement ou indirectement sur l'autre section de canule, sous l'effet d'une force exercée sur l'élément d'actionnement transversalement à l'axe longitudinal de la canule (100, 110, 120, 200, 400, 600, 700, 800), sachant que l'élément d'actionnement est un élément de levier qui est appuyable sur une section d'un élément d'appui (16) partant de l'autre section de canule par le biais d'une saillie (22) dépassant dudit élément d'actionnement.

2. Canule selon la revendication 1,
**caractérisée en ce**
**qu'**en cas de force exercée par l'élément d'actionnement dans le sens longitudinal de la canule (100, 110, 120, 200, 400, 600, 700, 800), de préférence en direction de la zone distale de la canule, la section de canule (10) d'où part l'élément d'actionnement est déplaçable tout d'abord dans le sens longitudinal de la canule le long de l'autre section de canule (12), et qu'ainsi sous l'effet prolongé de la force exercée, ladite section de canule est détachable de l'autre section de canule au moins dans la zone proximale.

3. Canule selon les revendications 1 et 2,
**caractérisée en ce**
**que** l'élément de levier (20, 25, 27) peut être appuyé sur une section en forme de rampe (26) de l'élément d'appui (16).

4. Canule selon au moins une des revendications précédentes,
**caractérisée en ce**
**que** l'élément d'appui (16) est un élément en forme de plaque s'étendant transversalement au sens longitudinal de la seconde section de canule (12), lequel élément entoure la première section de canule (10) dans le sens du détachement des sections de canule (10, 12) l'une de l'autre, avec un écartement qui permet un détachement des sections de canule.

5. Canule selon la revendication 1,
**caractérisée en ce**
**que** de la première section de canule (10) part un élément protecteur (30, 130) réglable le long du côté intérieur de celle-ci, qui est écarté de la pointe de ponction (32) dans la position d'utilisation de la canule et recouvre la pointe de ponction après la séparation des sections de canule.

6. Canule selon la revendication 5,
**caractérisée en ce**
**que** l'élément protecteur (30, 130) lors du déplacement longitudinal de la première section de canule (10) vers la seconde section de canule (12) est déplaçable en direction de la pointe de ponction (32) par interaction avec la seconde section de canule (12).

7. Canule selon au moins la revendication 5 ou 6,
**caractérisée en ce**
**que** l'élément protecteur (30) est un élément en forme de lamelle ou de gouttière dans le sens longitudinal duquel s'étend une fente (42) des côtés du bord longitudinal de laquelle partent des sections en forme d'ailettes (44, 46), que de la première section de canule (10) partent des sections de maintien (36, 38) recourbées vers l'intérieur, traversant la fente et reposant sur les côtés du bord longitudinal de celle-ci, qui présentent, dans le sens longitudinal de la première section de canule, une longueur qui est inférieure à la longueur de la fente et qu'au moins partiellement des sections du bord longitudinal (48, 50) de la seconde section de canule (12) reposent sur les bords longitudinaux (45, 47) des sections en forme d'ailettes, dans la position d'utilisation de la canule et que la seconde section de canule présente dans sa zone distale respectivement un évidement en forme de palier (60) dans sa zone de bord longitudinal, qui repose sur une des sections en forme d'ailettes pour permettre un mouvement relatif de l'élément protecteur par rapport à la première section de canule.

8. Canule selon au moins une des revendications 5 à 7,
**caractérisée en ce**
**que** la première section de canule (10) présente des trous oblongs (52, 54) s'étendant dans son sens longitudinal dans lesquels des saillies (56, 58) partant de l'élément protecteur (130) s'engagent, que la seconde section de canule (12) interagit avec l'élément protecteur de sorte que lors du déplacement longitudinal de la première section de canule vers la seconde section de canule, l'élément protecteur est déplaçable en direction de la pointe de ponction (32) pour recouvrir celle-ci.

9. Canule selon au moins une des revendications 5 à 8,
**caractérisée en ce**
**que** l'élément protecteur (130) présente du côté proximal une section en forme de palier (60) sur laquelle repose la zone distale de la seconde section de canule (12) lors du déplacement de la première section de canule (10) vers la seconde section de canule, ou que l'élément protecteur (130) présente dans sa zone proximale une lamelle (66) s'appuyant sur la paroi intérieure de la première section de canule (10) et destinée à appliquer une force dans la zone distale de l'élément protecteur en direction du côté intérieur de la première section de canule, et/ou que l'élément protecteur (130) présente dans sa zone distale un évidement (70) dans lequel s'engage la pointe de ponction (32) lorsque la pointe de ponction est recouverte, et/ou que l'élément protecteur (30) est coudé du côté distal, sachant que la pointe de ponction est positionnable à l'intérieur de la zone coudée (34).

10. Canule selon au moins une des revendications 5 à 9,
**caractérisée en ce**
**que** l'élément protecteur (30, 130) est conçu sous forme de lamelle ou de gouttière et repose côté extérieur à plat ou essentiellement à plat sur le côté intérieur de la première section de canule (10), et/ou que l'élément protecteur (30, 130) est constitué d'au moins un matériau du groupe des plastiques, métaux ou en contient.

11. Canule selon au moins une des revendications précédentes,
**caractérisée en ce**
**que** la première section de canule (10) présente perpendiculairement à l'axe longitudinal au moins partiellement une mesure d'arc B₁ telle que B₁ > π et que la seconde section de canule (12) présente une mesure d'arc B₂ telle que B₁ + B₂ > 2π et/ou que perpendiculairement à l'axe longitudinal de la canule, la première section de canule (10) présente dans la zone proximale une mesure d'arc B₃ et que la seconde section de canule (12) présente une mesure d'arc B4 telle que B3 < π et B3 + B4 > 2π, et/ou que la première et la seconde section de canule présentent dans la zone distale, notamment jusque dans la zone de l'élément protecteur (30, 130), respectivement une mesure d'arc B₅ telle que B₅ > π, notamment 1,2π < B₅ ≤ 1,75π.

12. Canule selon au moins une des revendications précédentes,
**caractérisée en ce**
**que** la première section de canule (10) présente une matière affaiblie, par ex. par estampage, sur le côté opposé à son ouverture longitudinale.

13. Canule selon au moins une des revendications précédentes,
**caractérisée en ce**
**qu'**une fente part du bord transversal proximal de la première section de canule (10) sur le côté opposé à son ouverture longitudinale et qu'un élément d'actionnement (25, 27) part de chacune des zones avoisinant la fente, de la première section de canule.

14. Canule selon au moins une des revendications précédentes,
**caractérisée en ce**
**qu'**au moins la première section de canule (10) présentant la pointe de ponction (20) est constituée de métal et que, le cas échéant, la première et la seconde section de canule (10, 12) sont constituées de matériaux différents, sachant que la seconde section de canule (12) est constituée de plastique et est, de préférence, une pièce moulée par injection.
